(19) Europäisches Patentamt
European Patent Office
Office européen des brevets

(11) Veröffentlichungsnummer: **0 379 917 B1**

(12) **EUROPÄISCHE PATENTSCHRIFT**

(45) Veröffentlichungstag der Patentschrift: **09.08.95**

(51) Int. Cl.$^{6}$: **C07C 233/49**, C07C 237/22, C07C 271/22, C07B 57/00, C08F 20/54

(21) Anmeldenummer: **90100703.9**

(22) Anmeldetag: **13.01.90**

Die Akte enthält technische Angaben, die nach dem Eingang der Anmeldung eingereicht wurden und die nicht in dieser Patentschrift enthalten sind.

(54) **Optisch aktive (Meth)Acrylsäure-Derivate, ihre Herstellung, ihre Polymerisation zu optisch aktiven Polymeren und deren Verwendung.**

(30) Priorität: **26.01.89 DE 3902287**

(43) Veröffentlichungstag der Anmeldung:
**01.08.90 Patentblatt 90/31**

(45) Bekanntmachung des Hinweises auf die Patenterteilung:
**09.08.95 Patentblatt 95/32**

(84) Benannte Vertragsstaaten:
**AT BE CH DE DK ES FR GB GR IT LI LU NL SE**

(56) Entgegenhaltungen:
**DE-A- 3 532 356**
**DE-A- 3 706 890**
**US-A- 4 473 690**

**CHEMICAL ABSTRACTS, Band 88, Nr. 26, 26. Juni 1978 Columbus, Ohio, USA H. BATZ et al. " Pharmacologically active polymers. Part 9. Slow-release forms of a morphine antagonist". Seite 399 Zusammenfassung-Nr. 197 559k**

(73) Patentinhaber: **BAYER AG**

**D-51368 Leverkusen (DE)**

(72) Erfinder: **Lange, Walter, Dr.**
**Auerstrasse 7**
**D-5000 Köln 60 (DE)**
Erfinder: **Bömer, Bruno, Dr.**
**Max-Planck-Strasse 53**
**D-5060 Bergisch-Gladbach 2 (DE)**
Erfinder: **Grosser, Rolf, Dr.**
**Gellertstrasse 9**
**D-5090 Leverkusen (DE)**
Erfinder: **Arlt, Dieter, Dr.-Prof.**
**Rybniker Strasse 2**
**D-5000 Köln 80 (DE)**

**EP 0 379 917 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 105, Nr. 18, 3. November 1986 Columbus, Ohio, USA S.MATSUGA et al. "Silver halide color photograhic material" Seite 613 Zusammenfassung-Nr. 162 148u

CHEMICAL ABSTRACTS, Band 105, Nr. 8, 25. August 1986, Columbus, Ohio, USA K.ULBRICH et al. "Polyethylene glycols containing enzy- matically degradablebonds" Seite 369, Zusammenfassung-Nr. 66 353k

Journal of High Resolution Chromatography & Chromatography Communications Vol.8 (1985), Seite 391-94

## Beschreibung

Die Erfindung betrifft neue optisch aktive N-(Meth)-Acryloyl-aminosäure-Derivate, ein Verfahren zu ihrer Herstellung, ihre Polymerisation zu optisch aktiven Polymeren und die Verwendung dieser optisch aktiven Polymere als Adsorbentien für die chromatographische Trennung von Racematen in ihre Enantiomere.

Die Auftrennung von Racematen in ihre optisch aktiven Komponenten hat in den letzten Jahren große Bedeutung gewonnen, weil festgestellt wurde, daß sich die optischen Antipoden biologisch aktiver Racemate wesentlich in ihrer biologischen Wirksamkeit unterscheiden und unterschiedliche Wirkungen und Nebenwirkungen aufweisen können. Es besteht daher ein großes Interesse an der Isolierung der einzelnen Enantiomeren biologisch wirksamer Racemate.

Für die chromatographische Trennung der Racemate wurden bereits die verschiedensten Adsorbentien vorgeschlagen; als günstigste Adsorptionsmittel haben sich dabei bislang die in Chem. Ber. 109 (1976), 1967-1975, den DE-A-25 00 523, 35 32 356, 36 19 303 und 37 06 890 beschriebenen polymeren (Meth)-acrylsäure-Derivate optisch aktiver Aminoverbindungen, gegebenenfalls in an Kieselgel gebundener Form erwiesen.

Bei der Anwendung der bekannten polymeren (Meth)acrylsäure-Derivate optisch aktiver Aminoverbindungen hat sich jedoch gezeigt, daß diese entweder nur eine unzureichende Wirkung aufweisen, dies ist z.B. der Fall bei den in den Chem. Ber. 109 loc.cit. und in der DE-A-36 19 303 beschriebenen Polymerisaten aus N-(Meth)-Acryloyl-aminosäureestern und den in der DE-A-25 00 523 beschriebenen Polymerisaten aus (Meth)Acrylamiden, oder sie weisen eine gute, aber nur auf bestimmte Racemate begrenzte Wirksamkeit auf, dies ist z.B. bei den in den DE-A-35 32 356 und 37 06 890 beschriebenen Polymerisaten der optisch aktiven Poly(meth)acrylamide der Fall, die sich von optisch aktiven N-(Meth)-Acryloyl-terpenylaminen ableiten.

In der Publikation von G. Schomburg et al., Journ. of High Chromatography & Chromatography Communications Vol. 8, (1985), 391-394 wird eine spezielle Verbindung im Zusammenhang mit Gaschromatographie beschrieben die der Formel I entspricht wenn $R^1$ Valin bedeutet. Es zeigt sich jedoch, daß gerade diese Acryloyl- Valin-Amid-Verbindung für die säulenchromatographische Trennung von Enantiomeren nicht so gut geeignet ist wie die beanspruchten Verbindungen.

Es wurde jetzt gefunden, daß man zu Adsorbentien mit wesentlich verbesserten Trenneigenschaften durch Polymerisation von optisch aktiven N-(Meth)Acryloylaminosäureestern und -amiden gelangt, deren Konformation infolge des sterischen Anspruchs der Ester- oder Amidgruppe in den optisch aktiven Aminosäureestern oder -amiden, gegebenenfalls unterstützt durch die Methylgruppe des Methacryloyl-Restes, soweit eingeschränkt ist, daß das Molekül des optisch aktiven N-(Meth)Acryloyl-aminosäure-Derivates insgesamt starr ist. Es wurde gefunden, daß die stark verbesserten Trenneigenschaften immer dann auftreten, wenn die Ester- oder Amid-Gruppen der optisch aktiven Aminosäure-Derivate einen sperrigen, d.h. raumerfüllenden Kohlenwasserstoff-Rest enthalten. Es wurde gefunden, daß die Raumerfüllung der tert.-Butylgruppe für sich allein noch nicht ausreicht, sondern daß diese erst in Kombination mit dem N-Methacryloylrest für eine Festlegung der Konformation des N-Methacryloyl-aminosäure-tert.-butylesters bzw. -amids ausreicht, während größervolumige Alkylreste, wie der Neopentylrest oder die verschiedenen Terpenylreste bereits für sich allein die Konformation hinreichend festlegen, unabhängig davon ob die Aminogruppe des optisch aktiven Aminosäure-Derivates an einen Acryloyl-oder Methacryloyl-Rest gebunden ist.

Die Versteifung des Moleküls der optisch aktiven N-(Meth)Acryloyl-aminosäurederivate führt zu einer wesentlichen Verbesserung der Trenneigenschaften der aus diesen optisch aktiven Aminosäurederivaten hergestellten Polymerisate.

An den erfindungsgemäßen Trennmaterialien können beispielsweise auch Tetra- und Hexahydrocarbazol- sowie Hydroxyalkylazol-Derivate gut getrennt werden, die an den bekannten Phasen nicht oder nur sehr unbefriedigend in die Enantiomere zerlegt werden können.

Die Erfindung betrifft daher optisch aktive N-(Meth)-Acryloyl-aminosäure-Derivate der Formel (I)

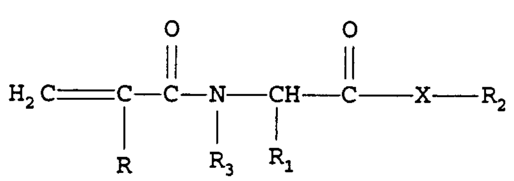

(I)

in welcher

- R Wasserstoff oder Methyl bedeutet,
- $R_1$ eine Alkylgruppe mit 1 bis 18 C-Atomen oder eine Cycloalkylgruppe mit 3 bis 8 C-Atomen bedeutet, die gegebenenfalls substituiert sind durch Hydroxy, Halogen, Alkoxy oder Cycloalkyl mit bis zu 8 Kohlenstoffatomen, durch eine Arylgruppe mit bis zu 14 Kohlenstoffatomen oder durch eine Heteroarylgruppe mit 4 bis 14 Kohlenstoffatomen, die 1 oder 2 Heteroatome aus der Gruppe Stickstoff, Sauerstoff oder Schwefel enthält, wobei die genannten Aryl- oder Heteroarylgruppen gegebenenfalls substituiert sind durch Hydroxy, Halogen, Alkyl oder Alkoxy mit jeweils 1 bis 4 C-Atomen,
- $R_3$ Wasserstoff bedeutet oder zusammen mit $R_1$ eine Tri- oder Tetramethylengruppe bedeutet,
- X Sauerstoff oder eine $NR_4$-Gruppe bedeutet, in der $R_4$ Wasserstoff ist oder für eine Alkylgruppe mit 1 bis 4 C-Atomen steht, oder in der $R_4$ zusammen mit $R_2$ und dem Stickstoffatom einen 5- bis 7-gliedrigen heterocyclischen Ring bildet, der gegebenenfalls mit einer COO-Alkylgruppe (1 bis 4 C-Atome) oder durch 1 oder 2 Alkylgruppen (jeweils 1 bis 4 C-Atome) substituiert ist, und
- $R_2$ einen sperrigen stark raumerfüllenden Kohlenwasserstoffrest mit bis zu 30 Kohlenstoffatomen oder einen Heteroarylrest mit 4 bis 14 Kohlenstoffatomen, der 1 Heteroatom aus der Gruppe Stickstoff, Sauerstoff oder Schwefel enthält, wobei die genannten Kohlenwasserstoff- und Heteroarylreste gegebenenfalls substituiert sind durch Halogen, Hydroxy, Alkyl und/oder Alkoxy mit jeweils 1 bis 8 Kohlenstoffatomen mit der Maßgabe, daß wenn $R_2$ eine tertiäre Butylgruppe ist oder X für den Rest $NR_4$ steht, R eine Methylgruppe sein muß.

Für $R^1$ seien als gegebenenfalls substituierte Alkyl-, Cycloalkyl-, Aralkyl-, Aryl- und Heteroaryl-Reste folgende Reste vorzugsweise genannt:

als gegebenenfalls sustituierte Alkylreste der Methyl-, Ethyl-, i-Propyl-, n-Propyl-, n-Butyl-, i-Butyl-, sec.-Butyl, tert.-Butyl, 1-Hydroxyethyl-, 2-Alkoxycarbonyl-,

3-Alkoxycarbonyl-, 3-N-Acylaminopropyl-, 4-N-Acylaminobutyl-, tert.-Butoxy-methyl- und der Hydroxymethyl-Rest;

als gegebenenfalls substituierte Cycloalkylreste der Cyclohexyl- und der Tetrahydronaphthyl-2-Rest;

als gegebenenfalls substituierte Aralkylreste der Benzyl- und 4-Hydroxybenzyl-Rest;

als gegebenenfalls substituierte Arylreste der Phenyl- und Naphthyl-Rest;

als gegebenenfalls substituierter Heteroarylrest der Indolyl-3-Rest.

Für $R_2$ seien als stark raumerfüllende Reste beispielsweise genannt:

tertiäre Alkylreste wie der tert.-Butyl-, der Neopentyl- und der Adamantyl-Rest;

in 1-Stellung durch Cycloalkylgruppen substituierte Alkylreste wie der Cyclohexylmethyl- oder Cyclopropylmethyl-Rest;

gegebenenfalls substituierte Cycloalkylreste wie der Cyclohexyl- und die durch Methyl- oder tert.-Butylgruppen substituierten Cyclohexyl-Reste wie der 2- oder 3-Methylcyclohexyl-, der 4-tert.-Butyl- und 2,6-Di-tert.-butyl-cyclohexyl- oder der Decahydronaphthylrest.

Aralkylreste wie der 1-Phenylethyl- und der 2-Phenylpropyl-Rest;

gegebenenfalls substituierte Phenylreste wie der Phenyl- oder durch $C_1$-$C_4$-Alkylgruppen substituierte Phenylreste wie der o-Tolyl-, 2,6-Xylyl-, 4-tert.-Butyl- und 2,6-Di-tert.-butyl-phenyl-Rest;

Terpenylreste wie der Menthyl-, Neomenthyl-, Bornyl-, Fenchyl- und Pinanyl-Rest.

Besonders vorteilhaft ist die Verwendung optisch aktiver Reste für $R_2$, z.B. des d- oder l-1-Phenylethyl- oder des d- oder l-Menthyl-, d- oder l-Neomenthyl-, d- oder l-Bornyl-, d- oder l-Fenchyl- oder des d- oder l-Pinanyl-Restes.

Die erfindungsgemäßen optisch aktiven N-(Meth)Acryloylaminosäure-Derivate der Formel (I) leiten sich vorzugsweise von optisch aktiven Aminosäuren wie Alanin, Aminobuttersäure, Valin, Norvalin, Leucin, Isoleucin, Terleucin, Phenylglycin, Phenylalanin, Naphthylananin, Cyclohexylglycin, Cyclohexylalanin, Tyrosin, Tryptophan, Threonin, Serin, Asparaginsäure, Glutaminsäure, Ornithin, Lysin oder Prolin ab.

Bevorzugte erfindungsgemäße optisch aktive N-(Meth)Acryloyl-aminosäure-Derivate der Formel (I) sind die N-Methacryloyl-Derivate der tert.-Butylester folgender Aminosäuren: Alanin, Valin, Leucin, Isoleucin, Phenylglycin, Cyclohexylglycin, Phenylalanin, Cyclohexylalanin, O-tert.-Butylserin, N'-Acyllysin z.B, N'-t-Butoxycarbonyllysin und N'-Acyl-ornithin; die N-(Meth)Acryloyl-Derivate der Bornyl-, Menthyl- und Fenchylester folgender Aminosäuren: Alanin, Phenylalanin, Cyclohexylalanin, Naphthylalanin, Phenylglycin, Cyclohexylglycin, Leucin, Isoleucin, Valin, Lysin, Ornithin; die N-(Meth)Acryloyl-Derivate der 4-tert.-Butyl-cyclohexylester folgender Aminosäuren: Alanin, Phenylalanin, Cyclohexylalanin, Phenylglycin, Cyclohexylglycin, Naphtyhlalanin, Leucin, Isoleucin und Valin;

die N-(Meth)Acryloyl-Derivate der 2-Decahydronaphthyl-ester folgender Aminosäuren: Alanin, Valin, Leucin, Isoleucin, Phenylglycin, Cyclohexylglycin, Phenylalanin, Cyclohexylalanin und Naphthylalanin; die N-(Meth)-Acryloyl-Derivate der Menthyl-, 1-Cyclohexylethyl- und 1-Phenylethyl-Amide folgender Aminosäuren: Alanin, Valin, Leucin, Isoleucin, Phenylglycin, Cyclohexylglycin, Phenylalanin, Cyclohexylalanin, Naphthylalanin und Prolin.

Besonders bevorzugte N-(Meth)Acryloyl-aminosäure-Derivate der Formel (I) sind: N-(Meth)-Acryloylalaninmenthylester, N-(Meth)Acryloylalanin-bornylester, N-(Meth)Acryloylalanin-fenchylester, N-(Meth)Acryloylphenylalanin-menthylester, N-Methacryloyl-phenylglycin-tert.-butylester, N-Methacryloyl-leucin-tert.-butylester, N-Methacryloyl-phenylalanin-tert.-butylester, N-(Meth)Acryloyl-valin-trans-4-tert.-butyl-cyclohexylester, N-Methacryloyl-N'-tert.-butoxycarbonyl-lysin-tert.-butylester, N-Methacryloyl-isoleucin-tert.-butyl-ester, N-Methacryloyl-valin-tert.-butylester, N-Methacryloyl-cyclohexylalanin-tert.-butylester, N-(Meth)-Acryloyl-alanin-2-decahydronaphthylester, N-Methacryloyl-alanin-menthylamid, N-Methacryloyl-phenyl-alanin-menthylamid und N-Methacryloylphenyl-alanin-1-phenylethylamid.

Die erfindungsgemäßen optisch aktiven N-(Meth)Acryloylaminosäure-Derivate der Formel (I) werden durch Umsetzung von optisch aktiven Aminosäure-Derivaten der Formel

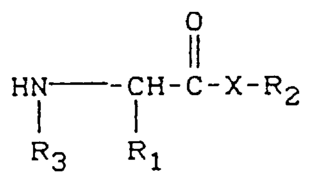

(II),

in der

$R_1$, $R_2$ $R_3$ und X  die unter Formel (I) angegebene Bedeutung haben,

oder deren Säureadditionsprodukten mit (Meth)Acrylsäure-Derivaten der Formel

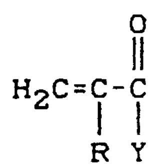

(III),

in der

Y  für eine abspaltbare Gruppe steht und

R  die unter Formel (I) angegebene Bedeutung hat,

gegebenenfalls in Gegenwart eines Säurebindemittels in inerten organischen Lösungsmitteln erhalten.

Als abspaltbare Gruppen seien genannt: Halogenatome, insbesondere Chlor oder Brom, oder eine Gruppe der Formel $OR_5$, in der $R_5$ eine $C_1$-$C_4$-Alkylgruppe oder eine $CH_2=C(R)$-COO-Gruppe (in der R die unter Formel (I) angegebene Bedeutung hat) darstellt.

Die Umsetzung kann bei normalem, erhöhtem oder bei erniedrigtem Druck durchgeführt werden. Im allgemeinen arbeitet man bei Normaldruck.

Die Verbindungen der Formeln (II) und (III) werden bevorzugt in äquimolaren Mengen eingesetzt.

Die als Ausgangsverbindungen verwendeten optisch aktiven Aminosäureester der Formel (II) und die Acrylsäurederivate der Formel (III) sind bekannt oder können nach an sich bekannten Verfahren hergestellt werden (siehe Bull. Chem. Soc. Jap. 37 (19), 191; EP-A-256 475; Beilsteins Handbuch der organischen Chemie, Band 2, 3. Ergänzungswerk, Seite 1293; Band 2, Hauptwerk, Seite 400).

Geeignete Säureadditionsverbindungen der als Ausgangsverbindungen zu verwendenden Aminosäuren sind Salze dieser Aminosäuren mit anorganischen oder organischen Säuren. Bevorzugt sind Mineralsäuren wie Salzsäure, Bromwasserstoffsäure, Schwefelsäure, Phosphorsäure oder organische Säuren wie Essigsäure, Methan-, Ethan-, Benzol- oder Toluolsulfonsäure.

Als Lösungsmittel eignen sich alle unter den Reaktionsbedingungen inerten organischen Lösungsmittel. Bevorzugt sind Kohlenwasserstoffe wie Benzol, Toluol, Xylol oder Erdölfraktionen, oder Halogenkohlenwasserstoffe wie Di-, Tri- oder Tetrachlormethan, Dichlorethan oder Trichlorethylen.

Als Säurebindemittel kommen vor allem die üblichen anorganischen oder organischen Basen in Betracht; bevorzugt werden Alkali-oder Erdalkalihydroxide wie Natrium-, Kalium-, Lithium-, Calcium- oder Bariumhydroxid, Alkali- oder Erdalkalicarbonate wie Natrium- oder Kaliumcarbonat, Alkalialkoholate wie

Natriummethylat, -ethylat, Kaliummethylat, -ethylat oder Amine wie Triethylamin oder Pyridin, verwendet.

Die Umsetzung der (Meth)Acrylsäure-Derivate der Formel (III) mit den Aminosäure-Derivaten der Formel (II) wird bei Temperaturen von -78 bis +100° C, bevorzugt von -10° C bis +60° C vorgenommen.

Die Erfindung betrifft weiterhin die durch Polymerisation der optisch aktiven N-(Meth)Acryloyl-aminosäure-Derivate der Formel (I) erhältlichen optisch aktiven Poly-(meth)acrylamide, die Struktureinheiten der Formel

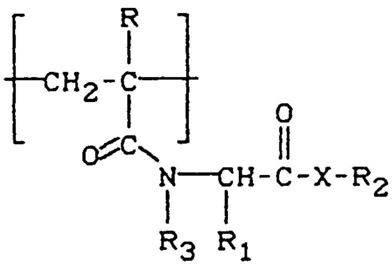

(IV)

enthalten, in der

R, $R_1$, $R_2$, $R_3$ und X die unter Formel (I) angegebene Bedeutung haben.

Die erfindungsgemäßen optisch aktiven Poly(meth)acrylamide liegen vorzugsweise in Form von vernetzten unlöslichen aber quellbaren Polymerisaten oder in an feinteilige anorganische Trägermaterialien gebundener Form vor. Sie können auch als lineare, in geeigneten organischen Lösungsmitteln lösliche Polymere hergestellt werden. Es ist ferner möglich, verschiedene erfindungsgemäße (Meth)-acrylamidmonomere cozupolymerisieren, sowie 0,1 bis 60, vorzugsweise 0,1 bis 20 Mol % copolymerisierbarer, nicht chiraler Monomere in das Polymere einzubauen.

Die vernetzten Polymerisate liegen vorzugsweise in Form feinteiliger Perlen mit 5 - 200 µm Teilchendurchmesser vor. Sie werden durch Suspensionspolymerisation der optisch aktiven (Meth)-acrylamidmonomere der Formel I mit 0,5 - 50 Mol-%, vorzugsweise 1 - 20 Mol-%, besonders bevorzugt 3 - 15 Mol-% (bezogen auf die Gesamtmenge [Mol] der eingesetzten Monomere) eines geeigneten Vernetzers in an sich bekannter Weise hergestellt.

Durch die Art und Menge des Vernetzers (der Vernetzer) läßt sich der Quellungsgrad der (Perl)-Polymerisate einstellen.

Bei der praktischen Verwendung haben sich (Perl)Polymerisate mit einem Quellungsgrad (Q) von 1.1 bis 10, bevorzugt 2.0 bis 7.0 bewährt.

$$Q = \frac{\text{Harzvolumen (gequollen)}}{\text{Harzvolumen (ungequollen)}}$$

Als Vernetzungsmittel kommen Verbindungen in Frage, die mindestens zwei polymerisierbare Vinylgruppen enthalten, Bevorzugte Vernetzungsmittel sind Alkandioldiacrylate wie 1,6-Hexandioldiacrylat, 1,4-Butandioldiacrylat, 1,3-Propandioldiacrylat oder 1,2-Ethylenglykoldiacrylat, oder Alkandioldimethacrylate wie 1,4-Butandioldimethacrylat, 1,3-Propandioldimethacrylat oder 1,2-Ethylenglykoldimethacrylat, aromatische Divinylverbindungen wie beispielsweise Divinylbenzol, Divinylchlorbenzol oder Divinyltoluol, Alkandicarbonsäurevinylester wie Adipinsäuredivinylester, Benzoldicarbonsäuredivinylester, Terephthalsäuredivinylester, N,N'-Alkylendiacrylamide wie N,N'-Methylendiacrylamid, N,N'-Ethylendiacrylamid, N,N'-Methylendimethacrylamid oder N,N'-Ethylendimethacrylamid.

Als Radikalbildner kommen die üblichen Radikalbildner in Frage. Bevorzugt sind Peroxide wie beispielsweise Dibenzoylperoxid, Dilauroylperoxid oder Di-orthotolylperoxid oder Azoverbindungen wie beispielsweise Azobisisobuttersäurenitril (AIBN). Auch Gemische verschiedener Radikalbildner sind verwendbar.

Die Polymerisationskomponenten werden in einem organischen Lösungsmittel, vorzugsweise einem aromatischen Kohlenwasserstoff wie Benzol oder Toluol, oder einem Halogenkohlenwasserstoff wie Di-, Tri-, Tetrachlormethan oder 1,2-Dichlorethan, gelöst.

Die organische Phase wird mit Hilfe eines wirksamen Rührers in der wäßrigen Lösung eines Schutzkolloids, bevorzugt in einer wäßrigen Lösung von Polyvinylalkohol, Polyvinylpyrrolidon oder eines Copolymers aus Methacrylsäure und Methylmethacrylat, gleichmäßig verteilt. Je Gewichtsteil organische Phase verwendet man etwa 1 bis 20, bevorzugt 2 bis 10 Gewichtsteile wäßrige Phase. Das Polymerisationsgemisch wird unter Rühren in einer Inertgasatmosphäre, vorzugsweise unter Stickstoff, auf Temperaturen von 30° C bis 100° C, vorzugsweise 40° C bis 80° C erhitzt. Die Polymerisationsdauer beträgt zwischen 2 und 24, bevorzugt 4 und 12 Stunden. Das auf diese Weise erhaltene Copolymerisat wird durch Filtration von der flüssigen Phase getrennt, durch gründliches Waschen mit Wasser und mit organischen Lösungsmitteln wie Methanol, Ethanol, Benzol, Toluol, Di-, Trichlormethan oder Aceton gereinigt und anschließend getrocknet.

Die Herstellung der erfindungsgemäßen optisch aktiven Poly(meth)acrylamide in an anorganische Trägermaterialien, vorzugsweise an Kieselgel, gebundener Form kann beispielsweise nach den in der DE-A 3 706 890 beschriebenen Verfahren erfolgen.

Breit anwendbar ist die Polymerisation der optisch aktiven (Meth)acrylamide in Gegenwart von Kieselgel-Diolphasen, die mit (Meth)acrylsäure verestert wurden. Die Polymerisation kann dabei in Abwesenheit von Lösungsmitteln oder in Gegenwart von Lösungsmitteln oder von Fällungsmitteln für die Poly(meth)-acrylamide durchgeführt werden. Als Initiatoren können die zur Herstellung der Perlpolymerisate verwendeten Radikalbildner ebenfalls eingesetzt werden.

Die polymermodifizierten Kieselgele werden intensiv mit Lösungsmitteln für das Polymere gewaschen und im Vakuum getrocknet.

Die Erfindung betrifft ferner die Verwendung der erfindungsgemäßen Poly-(meth)acrylamide als solche oder in vernetzter bzw. an Kieselgel gebundener Form zur chromatographischen Trennung von racemischen Gemischen in die optischen Antipoden. Besonders bewährt haben sich die erfindungsgemäßen Polymerisate zur chromatographischen Trennung von Tetrahydro- bzw. Hexahydrocarbazolderivaten wie z. B. 3-(4-Chlorphenylsulfonamido)-9-(2-carboxyethyl)-1,2,3,4,-tetrahydrocarbazol, 3-r-(Benzolsulfonamido)-9-(2-carboxyethyl)-1,2,3,4,4a,9a-hexahydrocarbazol, 1-(Carboxymethyl)-6-fluor-9-(4-chlorbenzyl)-1,2,3,4-tetrahydrocarbazol, Hydroxyalkylazol-derivaten wie z. B. 2-(4-Chlorphenyl)-3-methoxyimino-3-methyl-1-(1,2,4-triazolyl-1)-2-butanol und Erythro-1-(4-Chlorphenoxy)-1-(1,2,4-triazolyl-1)-3,3-dimethyl-2-butanol, Lactonderivate wie (E)-6α-[2-[3-(4-Fluorphenyl)-1-(1-methylethyl)-1H-indol-2-yl]-ethenyl]-tetrahydro-4β-hydroxy-2H-pyran-2-on, (E)-6α-[2-(3,5-Dichlor-4'-fluor-[1,1'-biphenyl]-2-yl)-ethenyl]-tetrahydro-4β-hydroxy-2h-pyran-2-on und 2-(4-Isobutylphenyl)-propionsäure.

Die Zusammensetzung des Fließmittels kann je nach Art und Eigenschaft des zu trennenden Racemates in üblicher Weise ausgewählt und optimiert werden. Die an Kieselgel gebundenen erfindungsgemäßen Poly-(meth)acrylamide können für chromatographische Racemattrennungen unter HPLC-Bedingungen eingesetzt werden.

Die Fähigkeit der Polymerisate zur Racemattrennung wird durch die Kapazitätsverhältnisse ($k'_{1(2)}$-Werte) für die beiden Enantiomere (1) und (2) und den daraus resultierenden Enantioselektivitätswert $\alpha$ ausgedrückt. Diese chromatographischen Parameter sind wie folgt definiert:

Kapazitätsverhältnis

$$k'_{1(2)} = \frac{t_{1(2)} - t_o}{t_o}$$

Enantioselektivität

$$\alpha = \frac{k'_2}{k'_1}$$

$t_o$ = Totzeit der Säule

$t_{1(2)}$ = Retentionszeit des zuerst eluierten Enantiomers 1 bzw. des später eluierten Enantiomers 2

Die präparative Trennung von racemischen Gemischen in ihre optischen Antipoden unter Verwendung der erfindungsgemäßen Polymerisate wird vorzugsweise säulenchromatographisch vorgenommen. Besonders vorteilhaft ist es hierfür, die chromatographische Trennung mit Perlpolymerisaten einer bestimmten Korngrößenverteilung vorzunehmen; gute Trennleistungen wurden mit Perlpolymerisaten einer Korngrößenverteilung von 5-200 µm, bevorzugt 15-100 µm erhalten.

Die Arbeitsmethodik der säulenchromatographischen Trennung ist bekannt. Üblicherweise wird das Polymerisat im Fließmittel suspendiert und die Suspension in eine Glassäule gefüllt. Nach Ablaufen des Fließmittels wird das zu trennende Racemat, gelöst in möglichst wenig Fließmittel, auf die Säule aufgebracht. Dann wird mit Fließmittel eluiert und die Enantiomeren im Eluat photometrisch und/oder polarimetrisch mittels geeigneter Durchflußzellen detektiert.

Als Fließmittel werden übliche organische Lösungsmittel bzw. Lösungsmittelgemische verwendet, die das als Adsorbens eingesetzte Polymerisat anquellen und das zu trennende Racemat lösen. Beispielsweise seien genannt: Kohlenwasserstoffe wie Benzol, Toluol oder Xylol, Ether wie Diethylether, Dioxan oder Tetrahydrofuran, Halogenkohlenwasserstoffe wie Di- oder Trichlormethan, Aceton, Acetonitril oder Essigester oder aber Gemische der genannten Lösungsmittel. Als besonders geeignet haben sich Mischungen aus Toluol und Tetrahydrofuran und aus Toluol und Dioxan erwiesen.

Beispiele

I Herstellung der optisch aktiven N-(Meth)Acryloylaminosäure-Derivate

Beispiel 1

Zur Lösung von 49,3 g (0,187 Mol) D-Alanin-l-menthylester-hydrochlorid in 750 ml Dichlormethan werden bei 0° C 39,4 g (0,39 Mol) Triethylamin zugegeben. Dann wird bei -10° C, eine Lösung von 17,2 g (0,19 Mol) Acrylsäurechlorid in 50 ml Dichlormethan zugetropft. Das Reaktionsgemisch wird 12 Stunden bei Raumtemperatur gerührt und anschließend mit Wasser, 5 %iger Salzsäure und abschließend mit gesättigter Natriumhydrogencarbonatlösung gewaschen. Die organische Phase wird über Magnesiumsulfat getrocknet. Nach dem Abdestillieren des Lösungsmittels wird der Rückstand aus Petrolether umkristallisiert.

Es werden 35,4 g N-Acryloyl-D-alanin-l-menthylester in Form farbloser Kristalle vom Fp. 79° C erhalten.

Drehwert $[\alpha]_D$: -67,0° (c = 1, $CHCl_3$)

Der Rückstand kann statt durch Kristallisation auch durch Chromatographie an Kieselgel unter Verwendung eines Ether/Petrolether-1:1-Gemisches als Laufmittel gereinigt werden.

Statt des D-Alanin-l-menthylester-hydrochlorides kann mit gleichem Erfolg auch der freie Aminoester eingesetzt werden.

Die Triethylaminmenge kann dann auf 0,19 Mol reduziert werden.

In der vorstehend für die Herstellung des N-Acryloyl-D-alanin-l-menthylesters beschriebenen Weise wurden aus den in Tabelle I und Ia angegebenen, optisch aktiven Aminosäure-Derivaten die ebenfalls in den Tabellen I und Ia angegebenen optisch aktiven N-(Meth)Acryloyl-aminosäure-Derivate erhalten. Die Ausbeuten, die Schmelzpunkte und die Drehwerte der erhaltenen Aminosäurederivate sind ebenfalls in den Tabellen angegeben.

T a b e l l e   I

N-A = N-Acryloyl-
N-Mea = N-Methacryloyl-

| Beispiel | verwendetes optisch aktives Aminosäure-Derivat | erhaltenes N-(Meth)Acryloyl-aminosäure-Derivat | Fp [°C] | $[\alpha]_D$ (c = 1, $CHCl_3$) | Ausbeute (% d.Th.) |
|---|---|---|---|---|---|
| 2 | L-Alanin-d-menthylester | N-A-L-alanin-d-menthylester | 79 | +67,2 | 70 |
| 3 | L-Alanin-l-menthylester | N-A-L-alanin-l-menthylester | 64 | -56,1 | 84 |
| 4 | D-Aminobuttersäure-l-menthylester | N-A-D-amino-buttersäure-l-menthylester | 72 | -64,2 | 78 |
| 5 | L-Valin-l-menthylester | N-A-L-valin-l-menthylester | 78 | +65,7 | 77 |
| 6 | D-Valin-l-menthylester | N-A-D-valin-l-menthylester | 72 | -58,4 | 80 |
| 7 | L-Norvalin-d-menthylester | N-A-L-norvalin-d-menthylester | 54-55 | +70,7 | 67 |
| 8 | L-Leucin-l-menthylester | N-A-L-leucin-l-menthylester | 72 | -18,0 | 90 |
| 9 | L-Leucin-d-menthylester | N-A-L-leucin-d-menthylester | 79 | +35,0 | 87 |
| 10 | D-Phenylglycin-l-menthylester | N-A-D-phenylglycin-l-menthylester | 130 | -176,5 | 75 |
| 11 | D-Phenylglycin-d-menthylester | N-A-D-phenylglycin-d-menthylester | 126 | +148,2 | 88 |
| 12 | L-Phenylalanin-d-menthylester | N-A-L-phenylalanin-d-menthylester | 79 | +97 | 78 |

T a b e l l e I (Fortsetzung)

N-A. = N-Acryloyl-
N-Mea = N-Methacryloyl-

| Beispiel | verwendetes optisch aktives Aminosäure-Derivat | erhaltenes N-(Meth)Acryloyl-aminosäure-Derivat | Fp [°C] | $[\alpha]_D$ (c = 1, $CHCl_3$) | Ausbeute (% d.Th.) |
|---|---|---|---|---|---|
| 13 | L-Alanin-d-menthylester | N-Mea-L-alanin-d-menthylester | 58 | +68,9 | 92 |
| 14 | L-Alanin-(+)-fenchylester | N-A-L-alanin-(+)-fenchylester | Öl | +29,6 | 84 |
| 15 | L-Phenylalanin-l-menthylester | N-A-L-phenylalanin-l-menthylester | 118 | -117,4 | 77 |
| 16 | L-Glutaminsäure-di-l-menthylester | N-A-L-glutaminsäure-di-l-menthylester | Öl | +78,1 | 60 |
| 17 | L-Leucin-tert.-butylester | N-Mea-L-leucin-tert.-butylester | 78 | +37,7 | 86 |
| 18 | D-Phenylglycin-tert.-butylester | N-Mea-D-phenylglycin-tert.-butylester | 118 | -125,0 | 74 |
| 19 | L-Phenylalanin-tert.-butylester | N-Mea-L-phenylalanin-tert.-butylester | 67 | +64,7 | 75 |
| 20 | N-ε-tert.-Butoxycarbonyl-L-lysin-tert.-butylester | α-N-Mea-N-ε-tert.-butoxy-carbonyl-L-lysin-tert.-butylester | 71 | +21,1 | 76 |
| 21 | D-Phenylglycin-cyclohexylester | N-Mea-D-phenylglycin-cyclohexylester | 62 | -85,9 | 85 |

T a b e l l e I (Fortsetzung)

N-A = N-Acryloyl-
N-Mea = N-Methacryloyl-

| Beispiel | verwendetes optisch aktives Aminosäure-Derivat | erhaltenes N-(Meth)Acryloyl-aminosäure-Derivat | Fp [°C] | $[\alpha]_D$ (c = 1, $CHCl_3$) | Ausbeute (% d.Th.) |
|---|---|---|---|---|---|
| 22 | L-Isoleucin-tert.-butylester | N-Mea-L-isoleucin-tert.-butyl ester | 63 | +52,6 | 84 |
| 23 | L-Leucin-cyclohexylester | N-Mea-L-leucin-cyclohexylester | 83 | +2,1 | 79 |
| 24 | L-Leucin-cyclohexylester | N-A-L-leucin-cyclohexylester | 105 | -3,9 | 74 |
| 25 | L-Alanin-l-bornylester | N-A-L-alanin-l-bornylester | 88 | -31,6 | 78 |
| 26 | L-Alanin-decahydronaphthyl-(2)-ester | N-A-L-alanin-decahydronaphthyl-(2)-ester | Öl | +2,2 | 77 |
| 27 | L-Valin-(trans-4-tert.-butyl-cyclohexyl)-ester | N-A-L-valin-(trans-4-tert.-butyl-cyclohexyl)-ester | 79 | +2,7 | 66 |

II Polymerisation der N-(Meth)Acryloyl-aminosäure-Derivate

1. Herstellung in Form von Perlpolymerisaten

Die Lösung von 13,5 g optisch aktivem N-(Meth)Acryloyl-aminosäure-Derivat, 1,5 g Ethylenglykoldimethacrylat und 0,3 g Azobisisobutyronitril in 37,5 g Trichlormethan wird unter Rühren (350 bis 500 U/min) in einer Lösung von 3 g Polyvinylalkohol in 130 ml entmineralisiertem Wasser dispergiert. Die Apparatur wird mehrmals evakuiert und mit Stickstoff gefüllt. Das Polymerisationsgemisch wird unter Stickstoff zunächst 30 Minuten bei Raumtemperatur und anschließend 16 Stunden bei 55° C (Innentemperatur) gerührt. Das Polymerisationsgemisch wird dann in 2 bis 3 l Wasser eingerührt und die flüssige Phase nach dem Absitzen des Perlpolymerisates dekantiert. Das Perlpolymerisat wird durch 3 bis 4-maliges Suspendieren in Wasser und Abdekantieren der flüssigen Phase vom Feinkorn (Polymerisat mit einer Korngröße von <10 µm) befreit und nach intensivem Waschen mit Aceton bei 60° C bis zur Gewichtskonstanz getrocknet.

In den nachstehenden Tabellen II und IIa sind die für die Polymerisation verwendeten N-(Meth)Acryloyl-aminosäure-Derivate, die Rührgeschwindigkeit, bei der die Polymerisation durchgeführt wurde, die Ausbeuten, in denen die Polymerisate erhalten wurden, deren Korngröße und das Volumen des erhaltenen Perlpolymerisates in trockenem ($V_s$) und gequollenem ($V_q$) Zustand (Quellmittel: Toluol) zusammengestellt:

**T a b e l l e   II**

| Beispiel Nr. | N-(Meth)Acryloyl-aminosäure-Derivat gemäß Beispiel | Rührgeschwindigkeit [U/min] | Ausbeute an Perlen >10 µm [g] | Korngröße der Perlen [µm] | $V_s$ [ml/g] | $V_q$ [ml/g] |
|---|---|---|---|---|---|---|
| 28 | 1 | 350 | 9,5 | 10-50 | 2,4 | 4,7 |
| 29 | 2 | 550 | 11,7 | 20-100 | 2,0 | 5,2 |
| 30 | 3 | 350 | 10,6 | 20-100 | 1,9 | 4,0 |
| 31 | 4 | 500 | 12,2 | 20-100 | 1,7 | 4,9 |
| 32 | 5 | 400 | 9,9 | 20-80 | 1,6 | 3,9 |
| 33 | 6 | 450 | 9,7 | 20-80 | 2,3 | 6,9 |
| 34 | 7 | 350 | 9,8 | 40-130 | 1,4 | 5,2 |
| 35 | 9 | 400 | 9,2 | 20-70 | 1,6 | 6,6 |
| 36 | 10 | 500 | 12,1 | 20-70 | 2,2 | 6,3 |
| 37 | 12 | 400 | 12,4 | 20-90 | 2,2 | 5,3 |
| 38 | 13 | 500 | 10,2 | 20-90 | 2,2 | 6,8 |
| 39 | 14 | 450 | 10,7 | 20-70 | 1,9 | 5,6 |
| 40 | 15 | 350 | 10,7 | 15-50 | 2,2 | 6,8 |
| 41 | 16 | 450 | 11,0 | 20-100 | 1,8 | 5,8 |
| 42 | 17 | 400 | 12,1 | 20-120 | 1,6 | 6,5 |
| 43 | 18 | 400 | 10,6 | 20-90 | 2,0 | 4,9 |
| 44 | 19 | 450 | 12,2 | 15-80 | 1,6 | 4,6 |

**Tabelle II (Fortsetzung)**

| Beispiel Nr. | N-(Meth)Acryloyl-aminosäure-Derivat gemäß Beispiel | Rührgeschwindigkeit [U/min] | Ausbeute an Perlen >10 µm [g] | Korngröße der Perlen [µm] | $V_s$ [ml/g] | $V_q$ [ml/g] |
|---|---|---|---|---|---|---|
| 45 | 20 | 500 | 11,3 | 15-70 | 1,8 | 3,8 |
| 46 | 21 | 450 | 12,0 | 15-60 | 1,6 | 4,5 |
| 47 | 25 | 450 | 11,4 | 20-100 | 1,7 | 6,0 |

2. Herstellung in an Kieselgel gebundener Form

a) 25 g mit 1,2-Diolgruppen modifiziertes Kieselgel (mittlere Korngröße: 5 µm) werden unter Feuchtigkeitsausschluß und unter Stickstoff in 500 ml Dioxan suspendiert. Die Suspension wird mit 16 ml Methacrylsäureanhydrid und 12,5 ml Triethylamin versetzt. Die Mischung wird 1 Stunde bei Raumtemperatur gerührt und 24 Stunden bei Raumtemperatur aufbewahrt. Dann wird das Kieselgel über eine Glasfritte (G4) abgesaugt, 3 x mit je 500 ml Dioxan 30 Minuten lang verrührt und zwischendurch gut trockengesaugt. Das durch Methacryloyl-Gruppen modifizierte Kieselgel wird bei Raumtemperatur im Vakuum ~ 0,005 at getrocknet.

Ausbeute 24,8 g

Elementaranalyse: C: 9,2 %, H: 1,7 %

Werte des mit Diolgruppen modifizierten Kieselgels: C: 7,7 %, H: 1,5 %

b) In einem mit Rückflußkühler und Magnetrührer versehenen 100 ml-Rundkolben werden 3 g des mit Methacryloylgruppen modifizierten Kieselgels, dessen Herstellung unter a) beschrieben ist, 6,0 g optisch aktives N-(Meth)Acryloyl-aminosäure-Derivat und 60 mg Azobisisobutyronitril in 25 ml trockenem Toluol gelöst bzw. suspendiert. Die Apparatur wird durch dreimaliges abwechselndes Evakuieren und mit Stickstoffüllen von Luft befreit und abschließend mit Stickstoff gefüllt. Das Polymerisationsgemisch wird 1 Stunde bei Raumtemperatur gerührt und dann rasch auf 80° C erhitzt. Nach 45-minütigem Rühren bei 80° C werden 200 mg 2,6-Di-tert.-butyl-4-methylphenol zugesetzt und das Reaktionsgemisch rasch abgekühlt. Das Kieselgel wird über eine Glasfritte (G4) abgesaugt, mit Toluol gewaschen und 2 x mit je 50 ml Chloroform, 1 x mit 50 ml Toluol und 1 x mit 50 ml Isopropanol je 30 Minuten verrührt und zwischendurch abgesaugt. Das Kieselgel wird abschließend bei Raumtemperatur im Vakuum ~ 0,005 at getrocknet. In den nachstehenden Tabellen III und IIIa sind die auf das modifizierte Kieselgel aufpolymerisierten N-(Meth)Acryloyl-aminosäure-Derivate, die Ausbeuten an optisch aktive Verbindungen enthaltendem Kieselgel, dessen Stickstoffgehalt und dessen Gehalt an gebundenem Polymer zusammengestellt.

c) 3,0 g optisch aktives N-(Meth)Acryloyl-aminosäure-Derivat werden in Chloroform gelöst (50-250 ml). Zu dieser Lösung werden 3,0 g des mit Methacryloylgruppen modifizierten Kieselgels nach 2a) zugegeben und das Chloroform bei 40° C Badtemperatur am Rotationsverdampfer vollständig entfernt, 60 mg Azobisisobutyronitril werden in 30 ml Methylenchlorid gelöst und zur Kieselgel-Monomermischung gegeben. Das Methylenchlorid wird bei 30° C und 0,02 at entfernt und die Apparatur anschließend sauerstofffrei gemacht und mit Stickstoff gefüllt. Die Mischung wird 3 Stunden auf 100° C erwärmt. Nach dem Abkühlen wied 3 x mit je 50 ml DMF, 1 x mit 50 ml THF und 2 x mit je 50 ml Isopropanol je 30 Minuten verrührt und zwischendurch abgesaugt.

Die Trocknung erfolgt wie beim Verfahren 2b). Die nach der Verfahrensweise 2c) hergestellten optisch aktiven Kieselgele sind in der Tabelle IIIa mit einem * gekennzeichnet.

**Tabelle III**

| Beispiel Nr. | N-(Meth)Acryloyl-aminosäure-Derivat gemäß Beispiel | Ausbeute [g] | [N-Gehalt] [%] | Gehalt des Kieselgels an gebundenem Polymerisat [Gew.-%] |
|---|---|---|---|---|
| 48 | 1 | 3,2 | 0,55 | 11,1 |
| 49 | 2 | 3,3 | 0,65 | 13,1 |
| 50 | 3 | 3,1 | 0,55 | 11,1 |
| 51 | 4 | 3,4 | 0,7 | 14,8 |
| 52 | 5 | 3,35 | 1,2 | 26,6 |
| 53 | 6 | 3,45 | 0,9 | 19,9 |
| 54 | 7 | 3,3 | 0,7 | 15,5 |
| 55 | 9 | 3,2 | 0,6 | 13,8 |
| 56 | 10 | 3,35 | 0,7 | 17,2 |
| 57 | 11 | 3,0 | 0,45 | 11,1 |
| 58 | 12 | 3,15 | 0,4 | 10,2 |
| 59 | 13 | 3,2 | 0,7 | 14,8 |
| 60 | 14 | 3,25 | 0,85 | 16,9 |
| 61 | 15 | 3,1 | 0,4 | 10,2 |
| 62 | 16 | 3,2 | 0,4 | 13,6 |
| 63 | 17 | 3,3 | 1,0 | 18,2 |
| 64 | 18 | 3,5 | 0,9 | 16,8 |

**Tabelle III** (Fortsetzung)

| Beispiel Nr. | N-(Meth)Acryloyl-aminosäure-Derivat gemäß Beispiel | Ausbeute [g] | [N-Gehalt] [%] | Gehalt des Kieselgels an gebundenem Polymerisat [Gew.-%] |
|---|---|---|---|---|
| 65 | 19 | 3,2 | 0,7 | 14,5 |
| 66 | 20 | 3,7 | 1,4 | 17,8 |
| 67 | 21 | 3,1 | 0,75 | 16,1 |
| 68 | 25 | 3,1 | 0,8 | 15,9 |
| 69 | 27 | 3,5 | 0,8 | 17,7 |

III Verwendung der optisch aktiven Polymerisate der N-(Meth)Acryloyl-aminosäure-Derivate als Adsorbentien für die Racemattrennung

Für die chromatographischen Trennungen wurden folgende Testracemate verwendet:

Racemat Nr. 1: 3-(4-Chlorphenylsulfonamido)-9-(2-carboxyethyl)-1,2,3,4-tetrahydrocarbazol
Racemat Nr. 2: 3-r-(Benzolsulfonamido)-9-(2-carboxyethyl)-1,2,3,4,4a,9a-hexahydrocarbazol
Racemat Nr. 3: 1-(Carboxylmethyl)-6-fluor-9-(4-chlorbenzyl)-1,2,3,4-tetrahydrocarbazol und
Racemat Nr. 4: 2-(4-Chlorphenyl)-3-methoximino-3-methyl-1-(1,2,3-triazolyl-1)-2-butanol
Racemat Nr. 5: (E)-6α[2-[3-(4-Fluorphenyl)-1-(1-methylethyl)-1H-indol-2-yl]ethenyl]tetrahydro-4β-hydroxy-2H-pyran-2-on
Racemat Nr. 6: (E)-6A[2-(3,5-Dichlor-4'-fluor[1,1'-biphenyl]-2-yl)ethenyl]tetrahydro-4β-hydroxy-2H-pyran-2-on und
Racemat Nr. 7: 2-(4-Isobutylphenyl)-propionsäure.

Die bei der chromatographischen Trennung der verschiedenen Testracemate 1 bis 4 mit Hilfe der erfindungsgemäßen Adsorbentien erhaltenen Ergebnisse (Enantioselektivität α und Kapazitätsverhältnis $k'_1$) und die verwendeten Elutionsmittel sind in der folgenden Tabelle IV zusammengestellt.

Die Perlpolymerisate wurden in einer Glassäule (Innendurchmesser: 1,2 cm; Betthöhe: 30-32 cm) eingesetzt. Eluiert wurde mit einem Toluol-Tetrahydrofuran-(2:1)-Gemisch (Elutionsmittel a) oder einem Toluol-Dioxan-(11:1)-Gemisch (Elutionsmittel b); die Fließmittelgeschwindigkeiten betrugen bei beiden Elutionsmitteln 0,5 ml/min.

Die an Kieselgel gebundenen Polymerisate wurden in Stahlsäulen (Innendurchmesser: 4 mm; Länge: 25 cm) eingesetzt. Eluiert wurde mit einem n-Heptan-Tetrahydrofuran-(2:1)-Gemisch (Elutionsmittel c), einem n-Heptan-Isopropanol-(10:1)-Gemisch (Elutionsmittel d) oder einem n-Heptan-Tetrahydrofuran-(10:1)-Gemisch (Elutionsmittel e); die Fließmittelgeschwindigkeit betrug 1 ml/min.

Tabelle IV

| Beispiel Nr. | Adsorbens gemäß Beispiel | Testracemat | Enantioselektivität α | Kapazitätsverhältnis $k'_1$ | Elutionsmittel |
|---|---|---|---|---|---|
| 70 | 28 | 1 | 1,33 | 1,43 | a |
| | | 2 | 1,84 | 0,84 | a |
| | | 4 | 1,23 | 0,62 | b |
| 71 | 36 | 1 | 1,36 | 0,41 | a |
| 72 | 37 | 1 | 1,71 | 0,40 | a |
| 73 | 39 | 1 | 1,78 | 1,73 | a |
| | | 2 | 2,42 | 0,69 | a |
| 74 | 42 | 1 | 1,61 | 1,75 | a |
| 75 | 43 | 1 | 1,43 | 1,00 | a |
| 76 | 48 | 2 | 1,38 | 2,62 | c |
| | | 3 | 1,18 | 1,43 | c |

**Tabelle IV** (Fortsetzung)

| Beispiel Nr. | Adsorbens gemäß Beispiel | Testracemat | Enantioselektivität α | Kapazitätsverhältnis $k_1$ | Elutionsmittel |
|---|---|---|---|---|---|
| 77 | 60 | 1 | 1,72 | 2,76 | c |
| | | 2 | 1,40 | 2,00 | c |
| 78 | 63 | 1 | 1,40 | 2,88 | c |
| | | 2 | 2,71 | 1,16 | c |
| | | 3 | 1,35 | 0,93 | c |
| 79 | 64 | 1 | 1,25 | 3,04 | c |
| | | 2 | 1,68 | 1,58 | c |
| 80 | 65 | 2 | 1,37 | 3,05 | c |
| 81 | 66 | 1 | 1,18 | 2,41 | c |
| 82 | 68 | 1 | 1,51 | 4,42 | c |
| | | 2 | 1,36 | 3,19 | c |
| 83 | 13 | 7 | 1,26 | 6,62 | e |

**Tabelle Ia**

N-A = N-Acryloyl

N-Mea = N-Methacryloyl-

| Beispiel | verwendetes optisch aktives Aminosäure-Derivat | erhaltenes N-(Meth)Acryloyl-aminosäure-Derivat | Fp. [°C] | $[\alpha]_D$ (c=1, $CHCl_3$) | Ausbeute (% der Theorie) |
|---|---|---|---|---|---|
| 84 | L-Alanin-t-butylester | N-Mea-L-Alanin-t-butylester | Kp.(0,6) 102 | + 37,4 | 90 |
| 85 | L-Alanin-(+)-fenchylester | N-Mea-L-Alanin-(+)-fenchylester | Öl | + 8,8 | 71 |
| 86 | D-Alanin-l-bornylester | N-A-D-Alanin-l-bornylester | Öl | - 23,0 | 60 |
| 87 | L-Valin-t-butylester | N-Mea-L-Valin-t-butylester | Öl | + 58,9 | 89 |
| 88 | L-Alanin-l-menthylamid | N-Mea-L-Alanin-l-menthylamid | 162 | -115,6 | 70 |
| 89 | L-Valin-t-butylamid | N-Mea-L-Valin-t-butylamid | 223 | + 4,5 | 50 |
| 90 | L-Leucin-l-bornylester | N-Mea-L-Leucin-l-bornylester | 100 | - 26,3 | 72 |
| 91 | L-Leucin-l-bornylester | N-A-L-Leucin-l-bornylester | 105 | -106,9 | 68 |
| 92 | L-Isoleucin-l-bornylester | N-A-L-Isoleucin-l-bornylester | Öl | - 5,3 | 55 |
| 93 | L-Isoleucin-l-bornylester | N-Mea-L-Isoleucin-l-bornylester | 105 | -106,9 | 71 |
| 94 | L-Phenylalanin-phenylester | N-A-L-Phenylalanin-phenylester | 122 | + 34,1 | 60 |

**Tabelle IIa**

| Beispiel Nr. | N-(Meth)acryloyl-aminosäure-Derivat gemäß Beispiel | Rührgeschwindigkeit [U/min] | Ausbeute an Perlen >10 µm [g] | Korngröße der Perlen [µm] | $V_s$ [ml/g] | $V_q$ [ml/g] |
|---|---|---|---|---|---|---|
| 95 | 84 | 450 | 10.8 | 25-90 | 1.7 | 5.7 |
| 96 | 88 | 450 | 11.1 | 20-100 | 2.2 | 5.5* |
| 97 | 90 | 450 | 11.4 | 25-110 | 2.0 | 6.9* |
| 98 | 91 | 450 | 10.8 | 20-90 | 1.6 | 5.1* |
| 99 | 92 | 450 | 9.2 | 20-100 | 1.9 | 7.0* |
| 100 | 93 | 450 | 9.8 | 20-90 | 2.0 | 7.9* |

* Quellungsmittel: Toluol-Tetrahydrofuran 3:2

Table IIIa

| Beispiel Nr. | N-(Meth)Acryloyl-aminosäure-Derivat gemäß Beispiel | Ausbeute [g] | [N-Gehalt] [%] | Gehalt des Kieselgels an gebundenem Polymerisat (Gew.-%) |
|---|---|---|---|---|
| 101 | 84 | 3.05 | 1.0 | 15.2 |
| 102 | 85 | 3.2 | 0.9 | 18.0 |
| 103 | 86 | 3.5 | 0.8 | 16.0 |
| 104 | 87 | 3.2 | 0.9 | 15.5 |
| 105 | 88 | 3.1 | 1.3 | 13.7 |
| 106 | 90 | 3.2 | 0.6 | 14.4 |
| 107 | 91 | 3.0 | 0.7 | 16.1 |
| 108 | 92 | 3.0 | 0.7 | 16.1 |
| 109 | 93 | 3.0 | 0.6 | 14.4 |
| 110 | 94 | 3.2 | 0.8 | 16.9 |

**Patentansprüche**

**Patentansprüche für folgende Vertragsstaaten : AT, BE, CH, DE, DK, FR, GB, IT, LI, LU, NL, SE**

1. Optisch aktive N-(Meth)-Acryloyl-aminosäure-Derivate der Formel

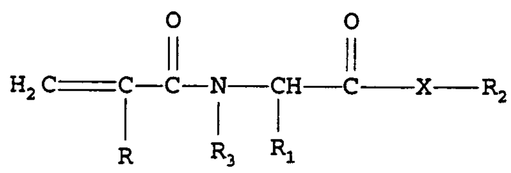

(I)

in welcher

- R Wasserstoff oder Methyl bedeutet,
- $R_1$ eine Alkylgruppe mit 1 bis 18 C-Atomen oder eine Cycloalkylgruppe mit 3 bis 8 C-Atomen bedeutet, die gegebenenfalls substituiert sind durch Hydroxy, Halogen, Alkoxy oder Cycloalkyl mit bis zu 8 Kohlenstoffatomen, durch eine Arylgruppe mit bis zu 14 Kohlenstoffatomen oder durch eine Heteroarylgruppe mit 4 bis 14 Kohlenstoffatomen, die 1 oder 2 Heteroatome aus der Gruppe Stickstoff, Sauerstoff oder Schwefel enthält, wobei die genannten Aryl- oder Heteroarylgruppen gegebenenfalls substituiert sind durch Hydroxy, Halogen, Alkyl oder Alkoxy mit jeweils 1 bis 4 C-Atomen,
- $R_3$ Wasserstoff bedeutet oder zusammen mit $R_1$ eine Tri- oder Tetramethylengruppe bedeutet,
- X Sauerstoff oder eine $NR_4$-Gruppe bedeutet, in der $R_4$ Wasserstoff ist oder für eine Alkylgruppe mit 1 bis 4 C-Atomen steht, oder in der $R_4$ zusammen mit $R_2$ und dem Stickstoffatom einen 5- bis 7-gliedrigen heterocyclischen Ring bildet, der gegebenenfalls mit einer COO-Alkylgruppe (1 bis 4 C-Atome) oder durch 1 oder 2 Alkylgruppen (jeweils 1 bis 4 C-Atome) substituiert ist, und
- $R_2$ einen sperrigen stark raumerfüllenden Kohlenwasserstoffrest mit bis zu 30 Kohlenstoffatomen oder einen Heteroarylrest mit 4 bis 14 Kohlenstoffatomen, der 1 Heteroatom aus der Gruppe Stickstoff, Sauerstoff oder Schwefel enthält, wobei die genannten Kohlenwasserstoff- und Heteroarylreste gegebenenfalls substituiert sind durch Halogen, Hydroxy, Alkyl und/oder Alkoxy mit jeweils 1 bis 8 Kohlenstoffatomen mit der Maßgabe, daß wenn $R_2$ eine tertiäre Butylgruppe ist oder X für den Rest $NR_4$ steht, R eine Methylgruppe sein muß.

2. Verbindungen der allgemeinen Formel I gemäß Anspruch 1, die abgeleitet sind von optisch aktiven Aminosäuren aus der Gruppe Alanin, Aminobuttersäure, Valin, Norvalin, Leucin, Isoleucin, Terleucin, Phenylglycin, Phenylalanin, Naphthylalanin, Cyclohexylglycin, Cyclohexylalanin, Tyrosin, Tryptophan, Threonin, Serin, Asparaginsäure, Glutaminsäure, Ornithin, Lysin oder Prolin.

3. Verfahren zur Herstellung von optisch aktiven N-(Meth)-Acryloyl-aminosäure-Derivaten der Formel

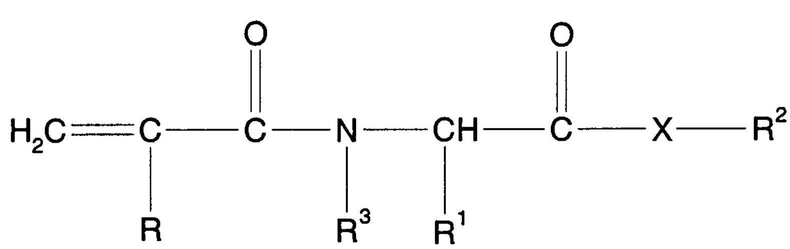

(I)

in welcher R, $R^1$, $R^2$, $R^3$ und X die in Anspruch 1 angegebene Bedeutung haben, dadurch gekennzeichnet, daß man optisch aktive Aminosäure-Derivate der Formel II

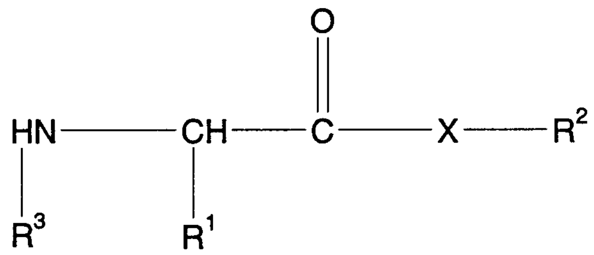

(II)

in welcher $R^1$, $R^2$, $R^3$ und X die unter Formel (I) angegebene Bedeutung haben, oder deren Säureadditionsprodukten mit (Meth)Acrylsäure-Derivaten der Formel

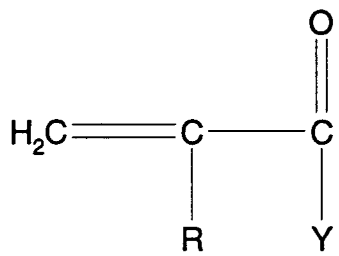

(III)

in welcher

- Y für eine abspaltbare Gruppe steht und
- R die unter Formel (I) angegebene Bedeutung hat,

gegebenenfalls in Gegenwart eines Säurebindemittels in inerten organischen Lösungsmitteln umsetzt.

**4.** Optisch aktive Poly-(meth)-acrylamide, enthaltend Struktureinheiten der Formel

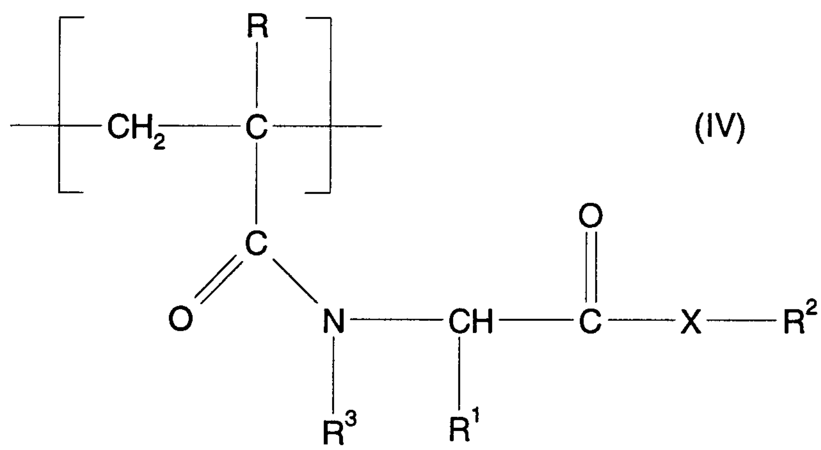

(IV)

in welcher

- R Wasserstoff oder Methyl bedeutet,
- $R_1$ eine Alkylgruppe mit 1 bis 18 C-Atomen oder eine Cycloalkylgruppe mit 3 bis 8 C-Atomen bedeutet, die gegebenenfalls substituiert sind durch Hydroxy, Halogen, Alkoxy oder Cycloalkyl mit bis zu 8 Kohlenstoffatomen, durch eine Arylgruppe mit bis zu 14 Kohlenstoffatomen oder durch eine Heteroarylgruppe mit 4 bis 14 Kohlenstoffatomen, die 1 oder 2 Heteroatome aus der Gruppe Stickstoff, Sauerstoff oder Schwefel enthält, wobei die genannten Aryl- oder Heteroarylgruppen gegebenenfalls substituiert sind durch Hydroxy, Halogen, Alkyl oder Alkoxy mit jeweils 1 bis 4 C-Atomen,
- $R_3$ Wasserstoff bedeutet oder zusammen mit $R_1$ eine Tri- oder Tetramethylengruppe bedeutet,
- X Sauerstoff oder eine $NR_4$-Gruppe bedeutet, in der $R_4$ Wasserstoff ist oder für eine Alkylgruppe mit 1 bis 4 C-Atomen steht, oder in der $R_4$ zusammen mit $R_2$ und dem Stickstoffatom

einen 5- bis 7-gliedrigen heterocyclischen Ring bildet, der gegebenenfalls mit einer COO-Alkylgruppe (1 bis 4 C-Atome) oder durch 1 oder 2 Alkylgruppen (jeweils 1 bis 4 C-Atome) substituiert ist, und

$R_2$ einen sperrigen stark raumerfüllenden Kohlenwasserstoffrest mit bis zu 30 Kohlenstoffatomen oder einen Heteroarylrest mit 4 bis 14 Kohlenstoffatomen, der 1 Heteroatom aus der Gruppe Stickstoff, Sauerstoff oder Schwefel enthält, wobei die genannten Kohlenwasserstoff- und Heteroarylreste gegebenenfalls substituiert sind durch Halogen, Hydroxy, Alkyl und/oder Alkoxy mit jeweils 1 bis 8 Kohlenstoffatomen mit der Maßgabe, daß wenn $R_2$ eine tertiäre Butylgruppe ist oder X für den Rest $NR_4$ steht, R eine Methylgruppe sein muß.

**5.** Optisch aktive Poly-(meth)-acrylamide der Formel IV gemäß Anspruch 4 in Form von quervernetzten oder linearen Polymeren.

**6.** Optisch aktive Poly(meth)-acrylamide der Formel IV gemäß Anspruch 4 in gebundener Form an feinverteiltem anorganischen Trägermaterial.

**7.** Verfahren zur Herstellung von optisch aktiven Poly-(meth)-acrylamiden der Formel IV gemäß Anspruch 4 durch übliche Polymerisation von optisch aktiven N-(Meth)-Acryloyl-aminosäure-Derivaten der Formel I gemäß Anspruch 1 in organischen Lösungsmitteln.

**8.** Verwendung von optisch aktiven Poly-(meth)-acrylamiden der Formel IV gemäß Anspruch 4 als solche, in quervernetzter Form und/oder in auf Silicagel gebundener Form zur säulenchromatographischen Trennung von racemischen Gemischen in ihre optischen Antipoden.

**9.** Verwendung der optisch aktiven Poly-(meth)-acrylamide gemäß Anspruch 8 für die säulenchromatographische Trennung von racemischen Gemischen von pharmakologischen Wirkstoffen.

**Patentansprüche für folgende Vertragsstaaten : ES, GR**

**1.** Verfahren zur Herstellung von optisch aktiven N-(Meth)-Acryloyl-aminosäure-Derivaten der Formel

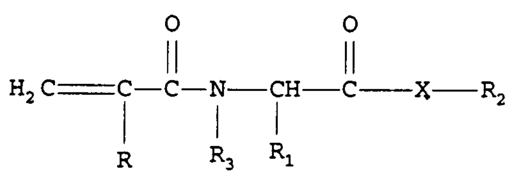

(I)

in welcher

R Wasserstoff oder Methyl bedeutet,

$R_1$ eine Alkylgruppe mit 1 bis 18 C-Atomen oder eine Cycloalkylgruppe mit 3 bis 8 C-Atomen bedeutet, die gegebenenfalls substituiert sind durch Hydroxy, Halogen, Alkoxy oder Cycloalkyl mit bis zu 8 Kohlenstoffatomen, durch eine Arylgruppe mit bis zu 14 Kohlenstoffatomen oder durch eine Heteroarylgruppe mit 4 bis 14 Kohlenstoffatomen, die 1 oder 2 Heteroatome aus der Gruppe Stickstoff, Sauerstoff oder Schwefel enthält, wobei die genannten Aryl- oder Heteroarylgruppen gegebenenfalls substituiert sind durch Hydroxy, Halogen, Alkyl oder Alkoxy mit jeweils 1 bis 4 C-Atomen,

$R_3$ Wasserstoff bedeutet oder zusammen mit $R_1$ eine Tri- oder Tetramethylengruppe bedeutet,

X Sauerstoff oder eine $NR_4$-Gruppe bedeutet, in der $R_4$ Wasserstoff ist oder für eine Alkylgruppe mit 1 bis 4 C-Atomen steht, oder in der $R_4$ zusammen mit $R_2$ und dem Stickstoffatom einen 5- bis 7-gliedrigen heterocyclischen Ring bildet, der gegebenenfalls mit einer COO-Alkylgruppe (1 bis 4 C-Atome) oder durch 1 oder 2 Alkylgruppen (jeweils 1 bis 4 C-Atome) substituiert ist, und

$R_2$ einen sperrigen stark raumerfüllenden Kohlenwasserstoffrest mit bis zu 30 Kohlenstoffatomen oder einen Heteroarylrest mit 4 bis 14 Kohlenstoffatomen, der 1 Heteroatom aus der Gruppe Stickstoff, Sauerstoff oder Schwefel enthält, wobei die genannten Kohlenwasserstoff- und Heteroarylreste gegebenenfalls substituiert sind durch Halogen, Hydroxy, Alkyl und/oder

Alkoxy mit jeweils 1 bis 8 Kohlenstoffatomen mit der Maßgabe, daß wenn $R_2$ eine tertiäre Butylgruppe ist oder X für den Rest $NR_4$ steht, R eine Methylgruppe sein muß,

dadurch gekennzeichnet, daß man optisch aktive Aminosäure-Derivate der Formel II

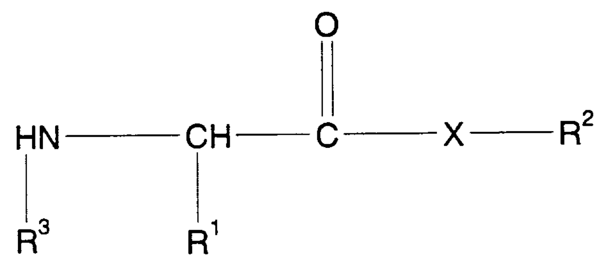

(II)

in welcher $R^1$, $R^2$, $R^3$ und X die unter Formel (I) angegebene Bedeutung haben,

oder deren Säureadditionsprodukten mit (Meth)Acrylsäure-Derivaten der Formel

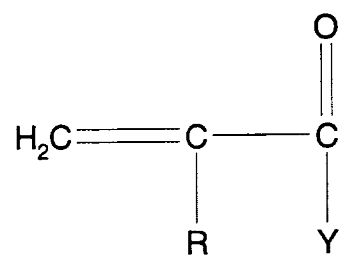

(III)

in welcher

Y für eine abspaltbare Gruppe steht und

R die unter Formel (I) angegebene Bedeutung hat,

gegebenenfalls in Gegenwart eines Säurebindemittels in inerten organischen Lösungsmitteln umsetzt.

**2.** Verfahren zur Herstellung von optisch aktiven Poly-(meth)-acrylamiden enthaltend Struktureinheiten der Formel IV

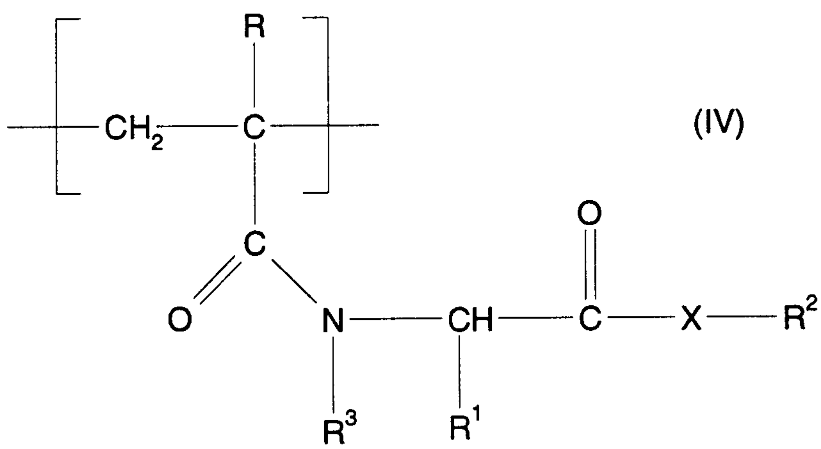

(IV)

in welcher

R Wasserstoff oder Methyl bedeutet,

$R_1$ eine Alkylgruppe mit 1 bis 18 C-Atomen oder eine Cycloalkylgruppe mit 3 bis 8 C-Atomen bedeutet, die gegebenenfalls substituiert sind durch Hydroxy, Halogen, Alkoxy oder Cycloalkyl mit bis zu 8 Kohlenstoffatomen, durch eine Arylgruppe mit bis zu 14 Kohlenstoffatomen oder durch eine Heteroarylgruppe mit 4 bis 14 Kohlenstoffatomen, die 1 oder 2 Heteroatome aus der Gruppe Stickstoff, Sauerstoff oder Schwefel enthält, wobei die genannten Aryl- oder

Heteroarylgruppen gegebenenfalls substituiert sind durch Hydroxy, Halogen, Alkyl oder Alkoxy mit jeweils 1 bis 4 C-Atomen,

$R_3$ Wasserstoff bedeutet oder zusammen mit $R_1$ eine Tri- oder Tetramethylengruppe bedeutet,

X Sauerstoff oder eine $NR_4$-Gruppe bedeutet, in der $R_4$ Wasserstoff ist oder für eine Alkylgruppe mit 1 bis 4 C-Atomen steht, oder in der $R_4$ zusammen mit $R_2$ und dem Stickstoffatom einen 5- bis 7-gliedrigen heterocyclischen Ring bildet, der gegebenenfalls mit einer COO-Alkylgruppe (1 bis 4 C-Atome) oder durch 1 oder 2 Alkylgruppen (jeweils 1 bis 4 C-Atome) substituiert ist, und

$R_2$ einen sperrigen stark raumerfüllenden Kohlenwasserstoffrest mit bis zu 30 Kohlenstoffatomen oder einen Heteroarylrest mit 4 bis 14 Kohlenstoffatomen, der 1 Heteroatom aus der Gruppe Stickstoff, Sauerstoff oder Schwefel enthält, wobei die genannten Kohlenwasserstoff- und Heteroarylreste gegebenenfalls substituiert sind durch Halogen, Hydroxy, Alkyl und/oder Alkoxy mit jeweils 1 bis 8 Kohlenstoffatomen mit der Maßgabe, daß wenn $R_2$ eine tertiäre Butylgruppe ist oder X für den Rest $NR_4$ steht, R eine Methylgruppe sein muß,

durch übliche Polymerisation von optisch aktiven N-(Meth)-Acryloyl-aminosäure-Derivaten der Formel I gemäß Anspruch 1 in organischen Lösungsmitteln.

**3.** Verwendung von optisch aktiven Poly-(meth)-acrylamiden der Formel IV gemäß Anspruch 2 als solche, in quervernetzter Form und/oder in auf Silicagel gebundener Form zur säulenchromatographischen Trennung von racemischen Gemischen in ihre optischen Antipoden.

**4.** Verwendung der optisch aktiven Poly-(meth)-acrylamide gemäß Anspruch 3 für die säulenchromatographische Trennung von racemischen Gemischen von pharmakologischen Wirkstoffen.

## Claims

**Claims for the following Contracting States : AT, BE, CH, DE, DK, FR, GB, IT, LI, LU, NL, SE**

**1.** Optically active N-(meth)-acryloyl-amino acid derivatives of the formula

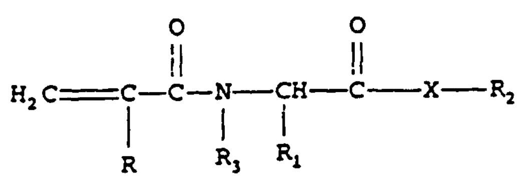

(I)

in which

R denotes hydrogen or methyl,

$R_1$ denotes an alkyl group having 1 to 18 C atoms or a cycloalkyl group having 3 to 8 C atoms, each of which is optionally substituted by hydroxyl, halogen, alkoxy or cycloalkyl having up to 8 carbon atoms, by an aryl group having up to 14 carbon atoms or by a heteroaryl group having 4 to 14 carbon atoms, which contains 1 or 2 heteroatoms from the group consisting of nitrogen, oxygen and sulphur, the aryl or heteroaryl groups mentioned optionally being substituted by hydroxyl, halogen, alkyl or alkoxy in each case having 1 to 4 C atoms,

$R_3$ denotes hydrogen or, together with $R_1$, denotes a tri- or tetramethylene group,

X denotes oxygen or an $NR_4$ group in which $R_4$ is hydrogen or represents an alkyl group having 1 to 4 C atoms or in which $R_4$, together with $R_2$ and the nitrogen atom, forms a 5- to 7-membered heterocyclic ring which is optionally substituted by a COO-alkyl group (1 to 4 C atoms) or by 1 or 2 alkyl groups (in each case 1 to 4 C atoms), and

$R_2$ denotes a bulky strongly space-filling hydrocarbon radical having up to 30 carbon atoms or a heteroaryl radical having 4 to 14 carbon atoms, which contains 1 heteroatom from the group consisting of nitrogen, oxygen and sulphur, the hydrocarbon and heteroaryl radicals mentioned optionally being substituted by halogen, hydroxyl, alkyl and/or alkoxy in each case having 1 to 8 carbon atoms, with the proviso that if $R_2$ is a tertiary butyl group or X represents the radical $NR_4$, R must be a methyl group.

**2.** Compounds of the general formula I according to Claim 1, which are derived from optically active amino acids from the group consisting of alanine, aminobutyric acid, valine, norvaline, leucine,

isoleucine, terleucine, phenylglycine, phenylalanine, naphthylalanine, cyclohexylglycine, cyclohexylalanine, tyrosine, tryptophan, threonine, serine, aspartic acid, glutamic acid, ornithine, lysine or proline.

**3.** Process for the preparation of optically active N-(meth)-acryloyl-amino acid derivatives of the formula

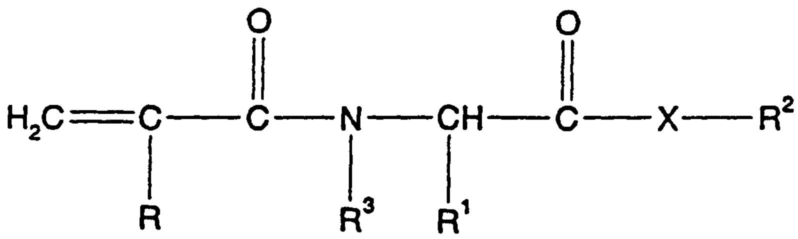

(I)

in which R, $R^1$, $R^2$, $R^3$ and X have the meaning specified in Claim 1,
characterized in that optically active amino acid derivatives of the formula II

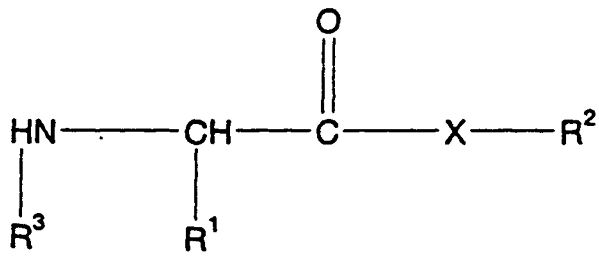

(II)

in which $R^1$, $R^2$, $R^3$ and X have the meaning specified under formula (I),
or their acid addition products are reacted in inert organic solvents with (meth)acrylic acid derivatives of the formula

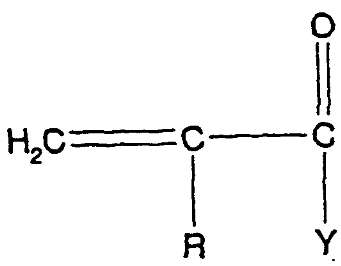

(III)

in which

Y represents a removable group and

R has the meaning specified under formula (I),

if appropriate in the presence of an acid-binding agent.

4. Optically active poly-(meth)acrylamides, containing structural units of the formula

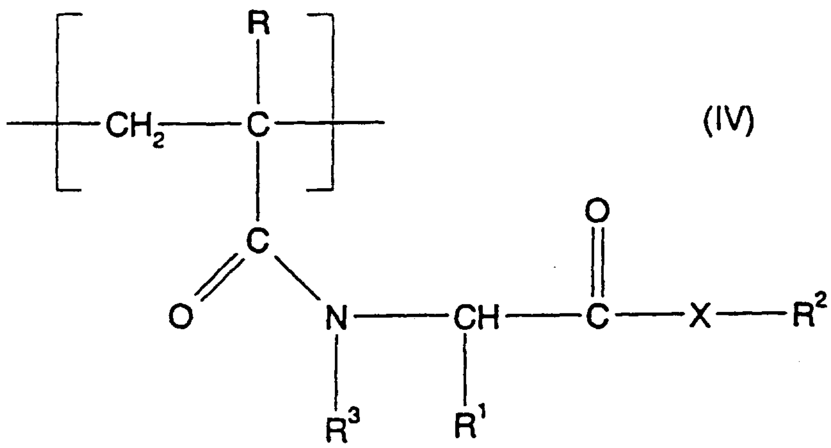

(IV)

in which

R denotes hydrogen or methyl,

$R_1$ denotes an alkyl group having 1 to 18 C atoms or a cycloalkyl group having 3 to 8 C atoms, each of which is optionally substituted by hydroxyl, halogen, alkoxy or cycloalkyl having up to 8 carbon atoms, by an aryl group having up to 14 carbon atoms or by a heteroaryl group having 4 to 14 carbon atoms, which contains 1 or 2 heteroatoms from the group consisting of nitrogen, oxygen and sulphur, the aryl or heteroaryl groups mentioned optionally being substituted by hydroxyl, halogen, alkyl or alkoxy in each case having 1 to 4 C atoms,

$R_3$ denotes hydrogen or, together with $R_1$, denotes a tri- or tetramethylene group,

X denotes oxygen or an $NR_4$ group in which $R_4$ is hydrogen or represents an alkyl group having 1 to 4 C atoms or in which $R_4$, together with $R_2$ and the nitrogen atom, forms a 5- to 7-membered heterocyclic ring which is optionally substituted by a COO-alkyl group (1 to 4 C atoms) or by 1 or 2 alkyl groups (in each case 1 to 4 C atoms), and

$R_2$ denotes a bulky strongly space-filling hydrocarbon radical having up to 30 carbon atoms or a heteroaryl radical having 4 to 14 carbon atoms, which contains 1 heteroatom from the group consisting of nitrogen, oxygen and sulphur, the hydrocarbon and heteroaryl radicals mentioned optionally being substituted by halogen, hydroxyl, alkyl and/or alkoxy in each case having 1 to 8 carbon atoms, with the proviso that if $R_2$ is a tertiary butyl group or X represents the radical $NR_4$, R must be a methyl group.

5. Optically active poly-(meth)-acrylamides of the formula IV according to Claim 4 in the form of crosslinked or linear polymers.

6. Optically active poly(meth)-acrylamides of the formula IV according to Claim 4 in bound form on finely divided inorganic support material.

7. A process for the preparation of optically active poly-(meth)-acrylamides of the formula IV according to Claim 4 by customary polymerization of optically active N-(meth-)acryloyl-amino acid derivatives of the formula I according to Claim 1 in organic solvents.

8. Use of optically active poly-(meth)-acrylamides of the formula IV according to Claim 4 as such, in crosslinked form and/or in silica gel-bound form for the separation of racemic mixtures into their optical antipodes by column chromatography.

9. Use of the optically active poly-(meth)-acrylamides according to Claim 8 for the separation of racemic mixtures of pharmacological active compounds by column chromatography.

**Claims for the following Contracting States : ES, GR**

1. Process for the preparation of optically active N-(meth)-acryloyl-amino acid derivatives of the formula

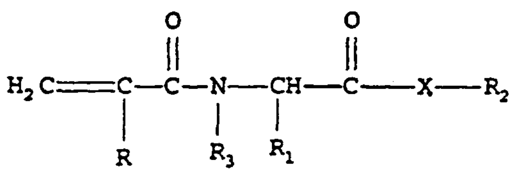

(I)

in which

R denotes hydrogen or methyl,

$R_1$ denotes an alkyl group having 1 to 18 C atoms or a cycloalkyl group having 3 to 8 C atoms, each of which is optionally substituted by hydroxyl, halogen, alkoxy or cycloalkyl having up to 8 carbon atoms, by an aryl group having up to 14 carbon atoms or by a heteroaryl group having 4 to 14 carbon atoms, which contains 1 or 2 heteroatoms from the group consisting of nitrogen, oxygen and sulphur, the aryl or heteroaryl groups mentioned optionally being substituted by hydroxyl, halogen, alkyl or alkoxy in each case having 1 to 4 C atoms,

$R_3$ denotes hydrogen or, together with $R_1$, denotes a tri- or tetramethylene group,

X denotes oxygen or an $NR_4$ group in which $R_4$ is hydrogen or represents an alkyl group having 1 to 4 C atoms or in which $R_4$, together with $R_2$ and the nitrogen atom, forms a 5- to 7-membered heterocyclic ring which is optionally substituted by a COO-alkyl group (1 to 4 C atoms) or by 1 or 2 alkyl groups (in each case 1 to 4 C atoms), and

$R_2$ denotes a bulky strongly space-filling hydrocarbon radical having up to 30 carbon atoms or a heteroaryl radical having 4 to 14 carbon atoms, which contains 1 heteroatom from the group consisting of nitrogen, oxygen and sulphur, the hydrocarbon and heteroaryl radicals mentioned optionally being substituted by halogen, hydroxyl, alkyl and/or alkoxy in each case having 1 to 8 carbon atoms, with the proviso that if $R_2$ is a tertiary butyl group or X represents the radical $NR_4$, R must be a methyl group,

characterized in that optically active amino acid derivatives of the formula II

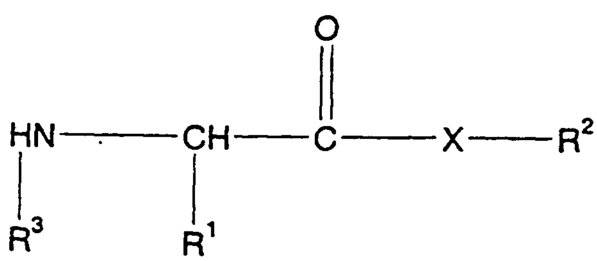

(II)

in which $R^1$, $R^2$, $R^3$ and X have the meaning specified under formula (I),
or their acid addition products are reacted in inert organic solvents with (meth)acrylic acid derivatives of the formula

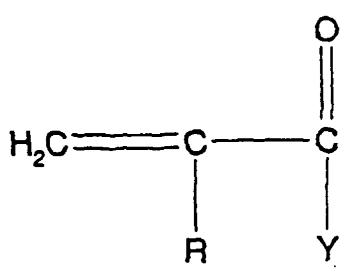

(III)

in which

Y represents a removable group and
R has the meaning specified under formula (I),
if appropriate in the presence of an acid-binding agent.

**2.** Process for the preparation of optically active poly-(meth)-acrylamides containing structural units of the formula IV

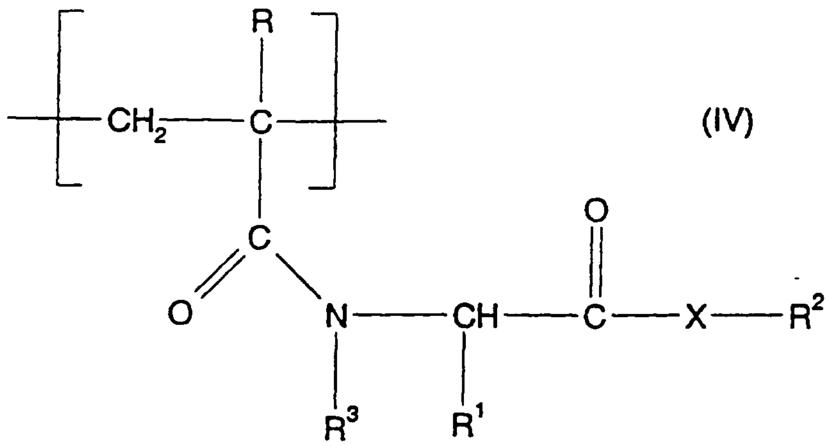

(IV)

in which
R denotes hydrogen or methyl,
$R_1$ denotes an alkyl group having 1 to 18 C atoms or a cycloalkyl group having 3 to 8 C atoms, each of which is optionally substituted by hydroxyl, halogen, alkoxy or cycloalkyl having up to 8 carbon atoms, by an aryl group having up to 14 carbon atoms or by a heteroaryl group having 4 to 14 carbon atoms, which contains 1 or 2 heteroatoms from the group consisting of nitrogen, oxygen and sulphur, the aryl or heteroaryl groups mentioned optionally being substituted by hydroxyl, halogen, alkyl or alkoxy in each case having 1 to 4 C atoms,
$R_3$ denotes hydrogen or, together with $R_1$, denotes a tri- or tetramethylene group,
X denotes oxygen or an $NR_4$ group in which $R_4$ is hydrogen or represents an alkyl group having 1 to 4 C atoms or in which $R_4$, together with $R_2$ and the nitrogen atom, forms a 5- to 7-membered heterocyclic ring which is optionally substituted by a COO-alkyl group (1 to 4 C atoms) or by 1 or 2 alkyl groups (in each case 1 to 4 C atoms), and
$R_2$ denotes a bulky strongly space-filling hydrocarbon radical having up to 30 carbon atoms or a heteroaryl radical having 4 to 14 carbon atoms, which contains 1 heteroatom from the group consisting of nitrogen, oxygen and sulphur, the hydrocarbon and heteroaryl radicals mentioned optionally being substituted by halogen, hydroxyl, alkyl and/or alkoxy in each case having 1 to 8 carbon atoms, with the proviso that if $R_2$ is a tertiary butyl group or X represents the radical $NR_4$, R must be a methyl group,

by customary polymerization of optically active N-(meth)-acryloyl-amino acid derivatives of the formula I according to Claim 1 in organic solvents.

**3.** Use of optically active poly-(meth)-acrylamides of the formula IV according to Claim 2 as such, in 'crosslinked form and/or in silica gel-bound form for the separation of racemic mixtures into their optical antipodes by column chromatography.

**4.** Use of the optically active poly-(meth)-acrylamides according to Claim 3 for the separation of racemic mixtures of pharmacological active compounds by column chromatography.

## Revendications

**Revendications pour les Etats contractants suivants : AT, BE, CH, DE, DK, FR, GB, IT, LI, LU, NL, SE**

**1.** Dérivés optiquement actifs de N-(méth)-acryloyl-aminoacides de formule :

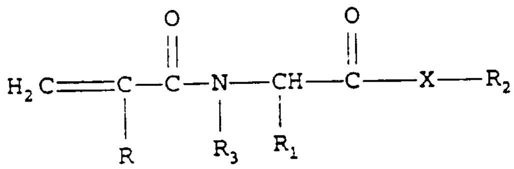

(I)

dans laquelle

- R représente l'hydrogène ou un groupe méthyle,
- $R_1$ est un groupe alkyle ayant 1 à 18 atomes de carbone ou un groupe cycloalkyle ayant 3 à 8 atomes de carbone, qui sont substitués le cas échéant par un radical hydroxy, halogéno, alkoxy ou cycloalkyle ayant jusqu'à 8 atomes de carbone, par un groupe aryle ayant jusqu'à 14 atomes de carbone ou par un groupe hétéroaryle ayant 4 à 14 atomes de carbone, qui contient un ou deux hétéroatomes du groupe azote, oxygène ou soufre, les groupes aryle ou hétéroaryle mentionnés étant éventuellement substitués par un radical hydroxy, un halogène ou un reste alkyle ou alkoxy ayant chacun 1 à 4 atomes de carbone,
- $R_3$ représente de l'hydrogène ou forme conjointement avec $R_1$ un groupe triméthylène ou tétraméthylène,
- X représente de l'oxygène ou un groupe $NR_4$ dans lequel $R_4$ est de l'hydrogène ou un groupe alkyle ayant 1 à 4 atomes de carbone, ou dans lequel $R_4$ forme conjointement avec $R_2$ et l'atome d'azote un noyau hétérocyclique pentagonal à heptagonal qui est substitué le cas échéant avec un groupe COO-alkyle (1 à 4 atomes de carbone) ou par un ou deux groupes alkyle (ayant chacun 1 à 4 atomes de carbone), et
- $R_2$ est un reste hydrocarboné de grand encombrement spatial ayant jusqu'à 30 atomes de carbone ou un reste hétéroaryle ayant 4 à 14 atomes de carbone, qui contient un hétéroatome du groupe azote, oxygène ou soufre, les restes hydrocarbonés et hétéroaryle mentionnés étant éventuellement substitués par un halogène, un radical hydroxy, un groupe alkyle et/ou un groupe alkoxy ayant chacun 1 à 8 atomes de carbone, sous réserve que lorsque $R_2$ est un groupe tertio-butyle ou X représente le reste $NR_4$, R soit alors impérativement un groupe méthyle.

**2.** Composés de formule générale I suivant la revendication 1, qui sont dérivés d'aminoacides optiquement actifs des groupes alanine, acide aminobutyrique, valine, norvaline, leucine, isoleucine, terleucine, phénylglycine, phénylalanine, naphtylalanine, cyclohexylglycine, cyclohexylalanine, tyrosine, tryptophane, thréonine, sérine, acide asparagique, acide glutamique, ornithine, lysine ou proline.

**3.** Procédé de production de dérivés optiquement actifs de N-(méth)-acryloyl-aminoacides de formule

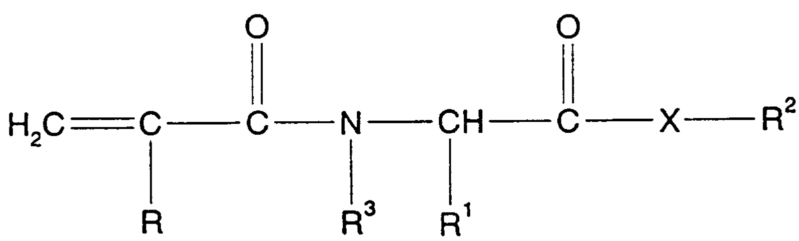

(I)

dans laquelle R, $R^1$, $R^2$, $R^3$ et X ont la définition indiquée dans la revendication 1,

caractérisé en ce qu'on fait réagir des dérivés d'aminoacides optiquement actifs de formule II

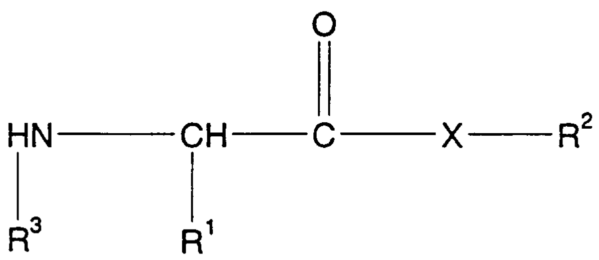

(II)

dans laquelle $R^1$, $R^2$, $R^3$ et X ont la définition indiquée pour la formule (I),

ou leurs produits d'addition d'acides avec des dérivés d'acide (méth)acrylique de formule

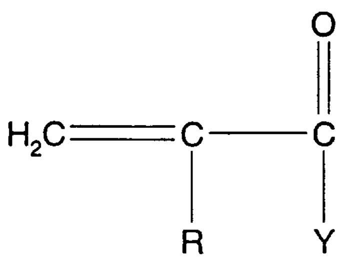

(III)

dans laquelle

Y est un groupe partant et

R a la définition indiquée pour la formule (I),

le cas échéant en présence d'un accepteur d'acide, dans des solvants organiques inertes.

**4.** Poly-(méth)acrylamides optiquement actifs, contenant des motifs structuraux de formule

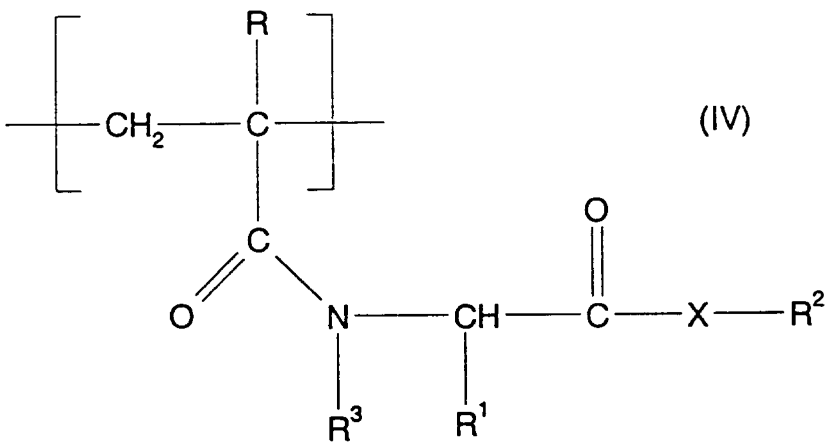

(IV)

dans laquelle

- R représente de l'hydrogène ou un groupe méthyle,
- $R_1$ est un groupe alkyle ayant 1 à 18 atomes de carbone ou un groupe cycloalkyle ayant 3 à 8 atomes de carbone, qui sont substitués le cas échéant par un radical hydroxy, halogéno, un groupe alkoxy ou cycloalkyle ayant chacun jusqu'à 8 atomes de carbone, par un groupe aryle ayant jusqu'à 14 atomes de carbone ou par un groupe hétéroaryle ayant 4 à 14 atomes de carbone, qui contient 1 ou 2 hétéroatomes du groupe azote, oxygène ou soufre, les groupes aryle ou hétéroaryle mentionnés étant substitués le cas échéant par un radical hydroxy, halogéno, un radical alkyle ou alkoxy ayant chacun 1 à 4 atomes de carbone,
- $R_3$ désigne de l'hydrogène ou forme conjointement avec $R_1$ un groupe triméthylène ou tétraméthylène,

X désigne de l'oxygène ou un groupe $NR_4$ dans lequel $R_4$ est de l'hydrogène ou un groupe alkyle ayant 1 à 4 atomes de carbone, ou dans lequel $R_4$ forme conjointement avec $R_2$ et l'atome d'azote un noyau hétérocyclique pentagonal à heptagonal, qui est substitué le cas échéant par un groupe COO-alkyle (1 à 4 atomes de carbone) ou par un ou deux groupes alkyle (ayant chacun 1 à 4 atomes de carbone), et

$R_2$ est un reste hydrocarboné de grand encombrement spatial ayant jusqu'à 30 atomes de carbone ou un reste hétéroaryle ayant 4 à 14 atomes de carbone, qui contient un hétéroatome du groupe azote, oxygène ou soufre, les restes hydrocarbonés et hétéroaryle mentionnés étant éventuellement substitués par un halogène, un radical hydroxy, un radical alkyle et/ou un radical alkoxy ayant chacun 1 à 8 atomes de carbone, sous réserve que lorsque $R_2$ est un groupe tertio-butyle ou bien X représente le reste $NR_4$, R soit impérativement un groupe méthyle.

**5.** Poly-(méth)acrylamides optiquement actifs de formule IV suivant la revendication 4, sous forme de polymères réticulés ou linéaires.

**6.** Poly(méth)-acrylamides optiquement actifs de formule IV suivant la revendication 4, sous la forme liée à un support inorganique finement divisé.

**7.** Procédé de production de poly(méth)-acrylamides optiquement actifs de formule IV suivant la revendication 4 par polymérisation classique de dérivés optiquement actifs de N-(méth)-acryloylaminoacides de formule I suivant la revendication 1 dans des solvants organiques.

**8.** Utilisation de poly-(méth)-acrylamides optiquement actifs de formule IV suivant la revendication 4 tels quels, sous la forme réticulée et/ou sous la forme liée à du gel de silice pour la séparation par chromatographie sur colonne de mélanges racémiques en leurs antipodes optiques.

**9.** Utilisation des poly-(méth)-acrylamides optiquement actifs suivant la revendication 8 pour la séparation chromatographique sur colonne de mélanges racémiques de substances douées d'activité pharmacologique.

**Revendications pour les Etats contractants suivants : GR, ES**

**1.** Procédé de production de dérivés optiquement actifs de N-(méth)-acryloylaminoacides de formule

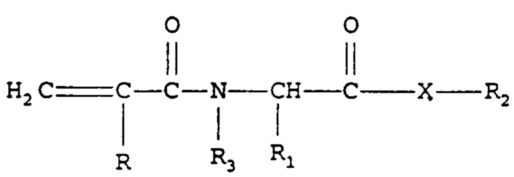

(I)

dans laquelle

R représente l'hydrogène ou un groupe méthyle,

$R_1$ est un groupe alkyle ayant 1 à 18 atomes de carbone ou un groupe cycloalkyle ayant 3 à 8 atomes de carbone, qui sont substitués le cas échéant par un radical hydroxy, halogéno, alkoxy ou cycloalkyle ayant jusqu'à 8 atomes de carbone, par un groupe aryle ayant jusqu'à 14 atomes de carbone ou par un groupe hétéroaryle ayant 4 à 14 atomes de carbone, qui contient un ou deux hétéroatomes du groupe azote, oxygène ou soufre, les groupes aryle ou hétéroaryle mentionnés étant éventuellement substitués par un radical hydroxy, un halogène ou un reste alkyle ou alkoxy ayant chacun 1 à 4 atomes de carbone,

$R_3$ représente de l'hydrogène ou forme conjointement avec $R_1$ un groupe triméthylène ou tétraméthylène,

X représente de l'oxygène ou un groupe $NR_4$ dans lequel $R_4$ est de l'hydrogène ou un groupe alkyle ayant 1 à 4 atomes de carbone, ou dans lequel $R_4$ forme conjointement avec $R_2$ et l'atome d'azote un noyau hétérocyclique pentagonal à heptagonal qui est substitué le cas échéant avec un groupe COO-alkyle (1 à 4 atomes de carbone) ou par un ou deux groupes

alkyle (ayant chacun 1 à 4 atomes de carbone), et

$R_2$ est un reste hydrocarboné de grand encombrement spatial ayant jusqu'à 30 atomes de carbone ou un reste hétéroaryle ayant 4 à 14 atomes de carbone, qui contient un hétéroatome du groupe azote, oxygène ou soufre, les restes hydrocarbonés et hétéroaryle mentionnés étant éventuellement substitués par un halogène, un radical hydroxy, un groupe alkyle et/ou un groupe alkoxy ayant chacun 1 à 8 atomes de carbone, sous réserve que lorsque $R_2$ est un groupe tertio-butyle ou X représente le reste $NR_4$, R soit alors impérativement un groupe méthyle,

caractérisé en ce qu'on fait réagir des dérivés d'aminoacides optiquement actifs de formule II

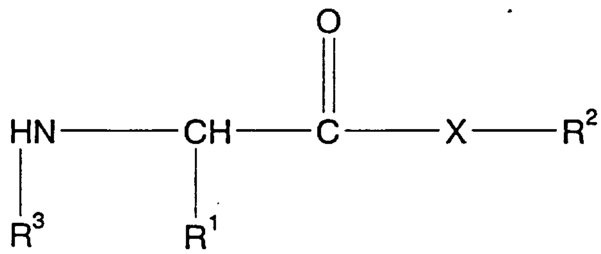

(II)

dans laquelle $R^1$, $R^2$, $R^3$ et X ont la définition indiquée pour la formule (I),

ou leurs produits d'addition d'acides avec des dérivés d'acide (méth)acrylique de formule

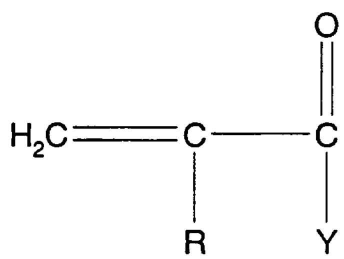

(III)

dans laquelle

Y est un groupe partant et

R a la définition indiquée pour la formule (I),

le cas échéant en présence d'un accepteur d'acide, dans des solvants organiques inertes.

**2.** Procédé de production de poly-(méth)-acrylamides optiquement actifs contenant des motifs structuraux de formule IV

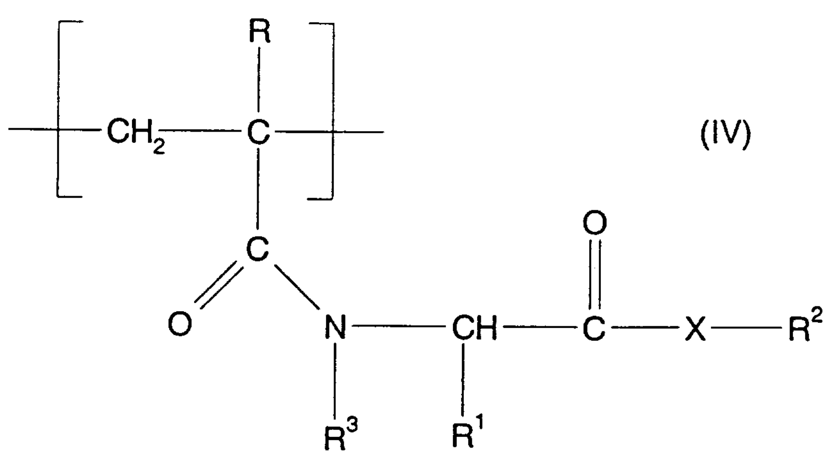

(IV)

dans laquelle

R représente de l'hydrogène ou un groupe méthyle,

$R_1$ est un groupe alkyle ayant 1 à 18 atomes de carbone ou un groupe cycloalkyle ayant 3 à 8 atomes de carbone, qui sont substitués le cas échéant par un radical hydroxy, halogéno, un groupe alkoxy ou cycloalkyle ayant chacun jusqu'à 8 atomes de carbone, par un groupe aryle ayant jusqu'à 14 atomes de carbone ou par un groupe hétéroaryle ayant 4 à 14 atomes de carbone, qui contient 1 ou 2 hétéroatomes du groupe azote, oxygène ou soufre, les groupes aryle ou hétéroaryle mentionnés étant substitués le cas échéant par un radical hydroxy, halogéno, un radical alkyle ou alkoxy ayant chacun 1 à 4 atomes de carbone,

$R_3$ désigne de l'hydrogène ou forme conjointement avec $R_1$ un groupe triméthylène ou tétraméthylène,

X désigne de l'oxygène ou un groupe $NR_4$ dans lequel $R_4$ est de l'hydrogène ou un groupe alkyle ayant 1 à 4 atomes de carbone, ou dans lequel $R_4$ forme conjointement avec $R_2$ et l'atome d'azote un noyau hétérocyclique pentagonal à heptagonal, qui est substitué le cas échéant par un groupe COO-alkyle (1 à 4 atomes de carbone) ou par un ou deux groupes alkyle (ayant chacun 1 à 4 atomes de carbone), et

$R_2$ est un reste hydrocarboné de grand encombrement spatial ayant jusqu'à 30 atomes de carbone ou un reste hétéroaryle ayant 4 à 14 atomes de carbone, qui contient un hétéroatome du groupe azote, oxygène ou soufre, les restes hydrocarbonés et hétéroaryle mentionnés étant éventuellement substitués par un halogène, un radical hydroxy, un radical alkyle et/ou un radical alkoxy ayant chacun 1 à 8 atomes de carbone, sous réserve que lorsque $R_2$ est un groupe tertio-butyle ou bien X représente le reste $NR_4$, R soit impérativement un groupe méthyle

par polymérisation classique de dérivés optiquement actifs de N-(méth)-acryloylaminoacides de formule I suivant la revendication 1 dans des solvants organiques.

**3.** Utilisation de poly-(méth)-acrylamides optiquement actifs de formule IV suivant la revendication 2 tels quels, sous la forme réticulée et/ou sous la forme liée à du gel de silice pour la séparation chromatographique sur colonne de mélanges racémiques en leurs antipodes optiques.

**4.** Utilisation des poly-(méth)-acrylamides optiquement actifs suivant la revendication 3 pour la séparation chromatographique sur colonne de mélanges racémiques de substances douées d'activité pharmacologique.